# EUROPÄISCHE PATENTANMELDUNG

(11) **EP 4 722 355 A1**
(43) Veröffentlichungstag der Anmeldung: **08.04.2026**
(21) Anmeldenummer: 24204230.7
(22) Anmeldetag: 02.10.2024
(51) Int. Cl.: C12N 9/02, C12P 17/04

(54) **VERFAHREN ZUR HERSTELLUNG VON 2,5-FURANDICARBONSÄURE**

(71) Anmelder: Annikki GmbH, 8074 Raaba-Grambach (AT); Treemera GmbH, 83064 Raubling (DE)
(72) Erfinder: Staunig, Nicole, 8074 Raaba-Grambach (AT); Dupont, Maria, 8074 Raaba-Grambach (AT)
(74) Vertreter: Schwarz & Partner Patentanwälte GmbH

(57) **Zusammenfassung**

Verfahren zur Herstellung von 5-Hydroxymethyl-2-furancarbonsäure (HMFA), indem 5-Hydroxymethylfurfural (HMF), welches in einer wässrigen Lösung vorliegt, durch Behandeln mit einer NAD(P)'-abhängigen Aldehyd-Dehydrogenase *in vitro* unter Bildung von NAD(P)H zu 5-Hydroxymethyl-2-furancarbonsäure (HMFA) oxidiert wird, wonach das bei der Oxidation entstandene NAD(P)H enzymatisch mit einer NAD(P)H-Oxidase wieder zu NAD(P)⁺ regeneriert wird, dadurch gekennzeichnet, dass die NAD(P)H-Oxidase eine Aminosäuresequenz umfasst, welche ausgewählt ist aus der Gruppe bestehend aus:
i) einer Aminosäuresequenz, die eine Identität zu SEQ ID Nr. 14, SEQ ID Nr. 16 oder SEQ ID Nr. 18 von mindestens 80% aufweist,
ii) einer Aminosäuresequenz, die durch eine Nukleinsäure kodiert wird, die eine Identität zu SEQ ID Nr. 13, SEQ ID Nr. 15 oder SEQ ID Nr. 17 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 13, SEQ ID Nr. 15 oder SEQ ID Nr. 17 bindet.

## Beschreibung

Die Erfindung betrifft ein Verfahren zur Herstellung von 2,5-Furandicarbonsäure (FDCA) aus 5-(Hydroxymethyl)furfural (HMF). Ferner betrifft die Erfindung ein Verfahren zur Herstellung von 5-Hydroxymethyl-2-furancarbonsäure (HMFA) aus 5-(Hydroxymethyl)furfural (HMF).

### Hintergrund der Erfindung

Im Jahr 2018 basierten in etwa 90% der produzierten Kunststoffe weltweit (400 Mt) auf fossilen Rohstoffen, die restlichen Anteile entfielen auf recycelte (9%), biobasierte (1%) und CO₂-basierte Kunststoffe (<1%) (Carus et al., 2020). Gleichzeitig wächst auch der Bedarf an Kunststoffen weltweit. 2019 lag die jährliche CO₂-Emission des gesamten Lebenszyklus von Kunststoffen bei 0,86 Gt, was einer CO₂-Emission von 189 Kohlekraftwerken, die mit voller Leistung (500 MW) laufen, entspricht. Für 2050 wird ein Anstieg auf 2,8 Gt (entspricht 615 Kohlenkraftwerken) prognostiziert (Hamilton et al., 2019).

Einer der Kunststoffe, die auf fossilen Rohstoffen basieren, ist Polyethylenterephthalat (PET), das vor allem in Form von Getränkeverpackungen Anwendung findet. Es handelt sich dabei ein Kondensationspolymer, welches aus Terephthalsäure (1,4-Benzoldicarbonsäure) und Ethylenglycol (Ethan-1,2-diol) unter Wasserabspaltung hergestellt wird.

Terephthalsäure wird großtechnisch durch Oxidation von *p*-Xylol (1,4-Dimethylbenzol) mit Luftsauerstoff bei ca. 200 °C in Gegenwart von Kobalt-Acetat, Mangan-Acetat und HBr in Essigsäure im sogenannten AMOCO-Prozess hergestellt (Tomäs et al., 2013).

Ethylenglycol wird durch die Hydrolyse von Ethylenoxid (bei 200 °C), welches wiederum durch die Oxidation von Ethen (gewonnen aus fossilen Rohstoffen) erhalten wird, hergestellt (Berger, 2016). Aufgrund der fossilen Quellen der Ausgangsstoffe (*p*-Xylol und Ethen) kann PET im Allgemeinen nicht als nachhaltig bezeichnet werden.

Um das 1,5-Grad-Ziel des Pariser Klimaabkommens zu erreichen und somit die negativen Folgen des Klimawandels zu begrenzen, müssen nachhaltige Alternativen für die petrochemisch basierten Kunststoffe gefunden werden. Für Ethylenglycol gibt es beispielsweise Verfahren, die auf erneuerbaren Ressourcen aufbauen. So kann beispielsweise Ethen auch durch die Dehydratisierung von Bio-Ethanol, der fermentativ aus Glucose oder Stärke erhalten wurde, hergestellt werden (z.B. Fan et al., 2013). Auch fermentative Umsetzungen von Xylose und/oder Glucose zu Ethylenglycol mittels metabolisch engineerten Mikroorganismen *(Escherichia coli* oder *Saccharomyces cerevisiae*) sind bekannt (Salusjärvi et al., 2019).

Der zweite Baustein von PET, die aromatische Verbindung Terephthalsäure, kann schwer aus nachhaltigen Rohstoffen hergestellt werden und muss daher ersetzt werden. Eine exzellente Substitution für die Terephthalsäure ist die 2,5-Furandicarbonsäure (FDCA), welche überwiegend aus dem katalytischen Upgrading von Biomasse erhalten wird. Durch die Polymerisation von FDCA mit Ethylenglycol entsteht PEF (Polyethylenfuranoat), das in der Struktur anstelle des Benzol-Rings einen heteroaromatischen Furan-Ring trägt. Wie PET ist PEF ein Thermoplast, zeichnet sich aber im Vergleich zu PET durch eine signifikant höhere Bioabbaubarkeit und bessere thermische (höhere Glasübergangstemperatur, niedrigere Schmelztemperatur) sowie mechanische Eigenschaften (höhere Steifheit) aus. Die wichtigste Eigenschaft von PEF aber ist die verringerte Permeabilität für Gase wie O₂ und CO₂. Dies ist besonders für Getränke wichtig, da dadurch die Haltbarkeit von Getränken (Verhinderung von Ausgasen karbonisierter Getränke; Verhinderung von Oxidationsprozessen durch diffundierten Sauerstoff) verlängert werden kann (de Jong et al., 2022).

Der mit Abstand wichtigste Ausgangsstoff für FDCA ist 5-(Hydroxymethyl)furfural (HMF), das beispielsweise aus Cellulose (und damit aus nachwachsenden Rohstoffen) gewonnen werden kann. Durch die enzymatische oder chemische Hydrolyse entsteht aus Cellulose D-Glucose, welche in weiterer Folge zu D-Fructose isomerisiert (enzymatisch oder chemisch) wird. Durch Dehydratisierung (Abspaltung von insgesamt drei H₂O-Molekülen) gelangt man von der D-Fructose zu HMF. Gängige Systeme zur Dehydratisierung von Fructose sind zum einen Mineralsäuren wie H₂SO₄ oder HCl und zum anderen (Brønsted- oder Lewis-)saure Feststoffkatalysatoren (Cong et al., 2021; US 9617234 B1).

HMF weist eine Alkohol- sowie eine Aldehyd-Funktion auf, die zu Carbonsäure-Gruppen oxidiert werden müssen, um FDCA zu erhalten. Die drei dazu notwendigen Oxidationsschritte können auf verschiedene Arten durchgeführt werden: chemisch mit heterogenen oder homogenen Katalysatoren, elektrochemisch und biokatalytisch (enzymatisch oder mit ganzen Zellen).

Ein Überblick über verschiedene chemische Synthesen findet sich im Artikel von Cong et al. (2021).

Biokatalytische Prozesse weisen gegenüber klassischen chemischen Verfahren einige Vorteile auf. Der Einsatz von Enzymen oder Zellen erlaubt beispielsweise hochselektive chemische Reaktionen unter milden Reaktionsbedingungen (wässeriges Milieu, Raumtemperatur, Umgebungsdruck), wobei die verwendeten Katalysatoren darüber hinaus auch bioabbaubar sind (Cong et al., 2021).

Eine Zwischenstufe in der Oxidation von HMF zu FDCA ist die 5-Hydroxymethyl-2-furancarbonsäure (HMFA), die beispielsweise als Baustein für Polymere wie Oligoester mit ε-Caprolacton als Komonomer (Todea et al., 2019) verwendet wird. Darüber hinaus weist HMFA cytotoxische und antitumorale Eigenschaften auf (Munekata & Tamura, 1981) und wirkt als Nematizid gegen die Fadenwürmer *Bursaphelenchus xylophilus* und *Caenorhabditis elegans* (Kimura et al., 2007).

Die Oxidation von HMF zu HMFA kann auf biokatalytischem Weg bewerkstelligt werden. So sind z.B. *resting cells* des Bakteriums *Deinococcus wulumuqiensis* R12 in der Lage, 511 mM HMFA mit einer Ausbeute von 85% und einer Produktivität von 44 g/(l·d) in 20 h in einem Fed-Batch-Verfahren (Zugabe von 0,75 mmol HMF alle 5 h) herzustellen. 2,5-Bis(hydroxymethyl)furan (BHMF) wurde als einziges Nebenprodukt in Spuren (< 1%) gebildet (Cang et al., 2019).

Aldehyd-Dehydrogenasen (ALDH) sind Enzyme, die die Oxidation von Aldehyd-Gruppen zu Carbonsäure-Gruppen katalysieren. Für den Organismus *Raoultella ornithinolytica* BF60 beispielsweise ist eine ALDH beschrieben, die HMF zu HMFA oxidieren kann (Hossain et al., 2017).

Zhang et al. testeten *Escherichia* coli-Ganzzell-Biokatalysatoren mit je einer exprimierten ALDH (Coniferylaldehyd-DH, Vanillin-DH 1, Vanillin-DH 2 oder Succinoylsemialdehyd-Pyridin-DH) aus *Comamonas testosteroni* SC1588 zur Oxidation von aromatischen und heteroaromatischen Aldehyden zu den entsprechenden Säuren. Für die Oxidation von HMF zu HMFA war die Vanillin-DH 1 am besten geeignet, wobei durch die zusätzliche Expression einer NADH-Oxidase (aus *Lactobacillus brevis*) die Ausbeuten gesteigert und die Bildung des Nebenprodukts BHMF reduziert werden konnte. Auf diese Weise konnten 250 mM HMF in 9 h mit einer Ausbeute von 95±2% zu HMFA oxidiert werden (Zhang et al., 2020).

In einer anderen Studie von Knaus et al. wurden drei verschiedene gereinigte Aldehyd-Dehydrogenasen (aus der Rinder-Augenlinse, *E. coli* sowie *Pseudomonas putida*) in Kombination mit einer NADH-Oxidase aus *Streptococcus mutans* zur Kofaktor-Regeneration auf 61 verschiedene aliphatische, arylaliphatische, benzylische, heteroaromatische und bizyklische Aldehyde getestet. Bei der Oxidation von 20 mM HMF konnten mit der ALDH aus der Rinder-Augenlinse in 4 h 90 % Ausbeute an HMFA und mit der ALDH aus *E. coli* in 24 h 91% Ausbeute erzielt werden. Die Reaktion wurde auf einen größeren Maßstab (2 g HMF) skaliert, wobei die *E. coli* ALDH in Form von lyophilisierten *E.* coli-Zellen zugegeben wurde (ohne NOX) und 1,37 g HMFA (61% Ausbeute) isoliert wurden (Knaus et al., 2018).

Die Kombination aus ALDH und NAD(P)H-Oxidase (kommerzielle NOX-009 von Prozomix, Ltd.) ist auch in US 10344307 B2 beschrieben, um 10 mM HMF mit 20 mol% Kofaktor (NAD⁺ oder NADP⁺) in 30 min vollständig zu HMFA zu oxidieren.

Qin et al. (2015) nutzten eine Xanthin-Oxidase (XO) aus *Escherichia coli,* ein Molybdän-abhängiges Enzym, um 26 mM HMF mit Sauerstoff zu HMFA zu oxidieren (94% Ausbeute in 7 h).

Ein weiterer Vertreter aus der Familie der Xanthin-Oxidasen, der Oxidation von HMF zu HMFA katalysiert, ist die periplasmatische Aldehyd-Oxidase (PaoABC) aus *E. coli* (McKenna et al., 2015; McKenna et al., 2017; US 10344307 B2).

Carro et al. (2015) beschrieben eine unspezifische Peroxygenase (UPO, EC 1.11.2.1; benötigt H₂O₂ als Oxidationsmittel) aus dem Pilz *Agrocybe aegerita,* welche 3 mM HMF in 24 h zu 97% zu HMFA oxidierte.

HMFA selbst kann enzymatisch entweder zu 5-Formyl-2-furancarbonsäure (FFA) oder FDCA oxidiert werden, wobei im Allgemeinen zwei verschiedene Enzyme für die einzelnen Oxidationsschritte (HMFA → FFA und FFA → FDCA) notwendig sind.

Mathieu et al. (2020) beschrieben die Oxidation von 10 mM HMFA zu FFA (46% Umsatz nach 16 h) mit einer Aryl-Alkohol-Oxidase (AAO) aus dem Pilz *Colletotrichum graminicola* in Gegenwart von Katalase und HRP (Meerrettich-Peroxidase).

In einer Studie von Cleveland et al. (2021) wurden zwei Aryl-Alkohol-Oxidasen aus den Pilzen *Fusarium graminearum* (*FgrAAO*) und *Fusarium oxysporum* (FoxAAO) charakterisiert. Diese konnten 10 mM HMFA (ebenfalls in Gegenwart von Katalase und HRP) zu FFA in 16,5 h umsetzen (Umsatz für FgrAAO: 84%, FoxAAO: 96%). Bei beiden Aryl-Alkohol-Oxidasen konnte keine Aktivität auf FFA festgestellt werden.

Cajnko et al. (2020) testeten eine Reihe von kommerziell erhältlichen Enzymen (Alkohol-Oxidase (AO) aus *Pichia pastoris*; Galactose-Oxidase aus *Dactylium dendroides*; Katalase aus *Aspergillus niger;* Laccase aus *Trametes versicolor;* eine pilzliche Lignin-Peroxidase (LPO) sowie HRP) auf 10 mM FFA und konnten nur für die AO, für die Laccase sowie für die LPO die Bildung von signifikanten Mengen FDCA (11,6% für AO, 1,1% für Laccase und 3,2% für LPO) nach 72 h beobachten. Als Nebenprodukt wurde HMFA gefunden (bis zu 18,2% für die AO). HMFA (10 mM) wurde in 72 h von der HRP oder LPO mit maximal 4,0% Umsatz zu FDCA (und max. 0,6% Umsatz zu FFA) umgesetzt.

Lappe et al. (2021) beschrieben eine UPO aus *Moesziomyces antarcticus,* welche 2 mM HMFA in 144 h komplett zu FFA (99,2%) und FDCA (0,8%) oxidierte. 2 mM FFA wiederum konnten in 144 h zu 40% FDCA umgesetzt werden.

Die von Carro et al. (2015) verwendete UPO *aus A. aegerita* katalysiert auch die Oxidation von FFA (3 mM) zu FDCA (90% Umsatz nach 120 h).

Jia et al. (2017) nutzten ein Enzymsystem bestehend aus Pferdeleber-Alkoholdehydrogenase (HLADH) und humanem Hämoglobin (oxidiert NADH mittels H₂O₂ zu NAD⁺), um 96% des Substrats (10 mM FFA) in 60 h zu FDCA oxidieren.

US 8183020 B2 beschreibt die enzymatische Oxidation von FFA zu FDCA mit einer kommerziell erhältlichen Chlorperoxidase aus *Caldariomyces fumago* (EC 1.11.1.10) mit H₂O₂ als Oxidationsmittel.

McKenna et al. (2017) verwendeten eine Kombination aus PaoABC und der Galactose-Oxidase M₃₋₅ (GOase M₃₋₅) in Kombination mit Katalase und HRP zur Oxidation von HMF zu FDCA, wobei die Oxidation von HMF zu 2,5-Diformylfuran (DFF) und von HMFAzu FFA von der GOase M₃₋₅ und die restlichen Schritte von der PaoABC katalysiert werden. Die Zugabe von HRP als Aktivator für die GOase M₃₋₅ ist dabei unerlässlich, da ansonsten die Oxidation von HMFA zu FFA nur sehr langsam abläuft und somit einen Flaschenhals der Kaskade darstellt.

Die hier vorgestellten Verfahren zur Herstellung von HMFA und FDCA weisen im Allgemeinen Nachteile wie niedrige Substratkonzentrationen, lange Reaktionszeiten oder unwirtschaftlich hohe Mengen an zugesetztem Kofaktor auf.

Hier setzt nun die Aufgabe der vorliegenden Erfindung an und setzt sich zum Ziel, verbesserte Verfahren zur Herstellung von HMFA und FDCA zur Verfügung zu stellen, die sich durch hohe Substratkonzentrationen und/oder hohe Umsätze auszeichnen.

### Detaillierte Beschreibung der Erfindung

Die Aufgabe zur Herstellung von 5-Hydroxymethyl-2-furancarbonsäure (HMFA) wird erfindungsgemäß gelöst, indem 5-Hydroxymethylfurfural (HMF), welches in einer wässrigen Lösung vorliegt, durch Behandeln mit einer NAD(P)⁺-abhängigen Aldehyd-Dehydrogenase *in vitro* unter Bildung von NAD(P)H zu 5-Hydroxymethyl-2-furancarbonsäure (HMFA) oxidiert wird, wonach das bei der Oxidation entstandene NAD(P)H enzymatisch mit einer NAD(P)H-Oxidase wieder zu NAD(P)⁺ regeneriert wird, dadurch gekennzeichnet, dass die NAD(P)H-Oxidase eine Aminosäuresequenz umfasst, welche ausgewählt ist aus der Gruppe bestehend aus:
i) einer Aminosäuresequenz, die eine Identität zu SEQ ID Nr. 14, SEQ ID Nr. 16 oder SEQ ID Nr. 18 von mindestens 80% aufweist,
ii) einer Aminosäuresequenz, die durch eine Nukleinsäure kodiert wird, die eine Identität zu SEQ ID Nr. 13, SEQ ID Nr. 15 oder SEQ ID Nr. 17 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 13, SEQ ID Nr. 15 oder SEQ ID Nr. 17 bindet.

Es hat sich überraschenderweise gezeigt, dass die NADH-Oxidasen mit den SEQ ID Nr. 14, 16 und 18 zu wesentlich höheren Umsätzen im Vergleich zu den im Stand der Technik beschriebenen NADH-Oxidasen *aus Streptococcus mutans* (Knaus et al., 2018) oder NOX-009 (US 10344307 B2) führen (siehe Beispiele 2 und 3).

Die Aufgabe zur Herstellung von 2,5-Furandicarbonsäure (FDCA) wird erfindungsgemäß gelöst, indem der wässerigen Lösung nach Bildung von 5-Hydroxymethyl-2-furancarbonsäure (HMFA) eine 5-Hydroxymethylfurfural-Oxidase (HMFO) zugegeben wird.

Es hat sich gezeigt, dass durch die Zugabe von HMFO die Oxidation von HMF nicht bei HMFA stoppt, sondern die Oxidation bis zur entsprechenden Dicarbonsäure (FDCA) durchläuft, da die NAD(P)⁺-abhängige Aldehyd-Dehydrogenase auch den letzten Oxidationsschritt von 5-Formyl-2-furancarbonsäure (FFA) zu FDCA katalysiert.

In einer weiteren Variante des erfindungsgemäßen Verfahrens wird der wässerigen Lösung auch eine Katalase zugegeben.

In einer weiteren bevorzugten Variante des erfindungsgemäßen Verfahrens wird das bei der Oxidation entstandene NAD(P)H zumindest zum Teil enzymatisch mit einer Oxidoreduktase wieder zu NAD(P)⁺ regeneriert und als Kosubstrat der Oxidoreduktase eine Keto-Verbindung eingesetzt.

Als Oxidoreduktasen werden bevorzugt Alkohol-Dehydrogenasen, Xylitol-Dehydrogenasen, Sorbitol-Dehydrogenasen, Xylose-Reduktasen oder SDR-Familie-Oxidoreduktasen eingesetzt.

Als Keto-Verbindung wird bevorzugt eine Ketose, insbesondere bevorzugt D-Fructose, eine Aldose, insbesondere bevorzugt D-Glucose oder D-Xylose, oder ein Keton, noch bevorzugter ein aliphatisches Keton und insbesondere bevorzugt Aceton, eingesetzt.

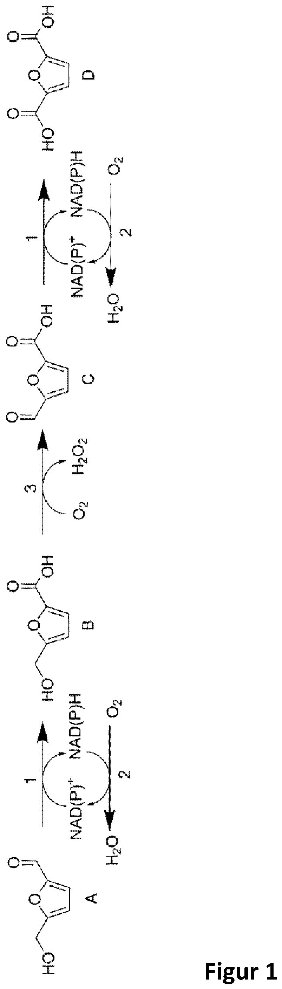

Bevorzugte Ausführungsformen des erfindungsgemäßen Verfahrens sind in der beiliegenden Figur 1 schematisch dargestellt. Die Bezeichnung A steht hierbei für 5-(Hydroxymethyl)furfural (HMF), B für 5-Hydroxymethyl-2-furancarbonsäure (HMFA), C für 5-Formyl-2-furancarbonsäure (FFA) und D für 2,5-Furandicarbonsäure (FDCA), 1 für Aldehyd-Dehydrogenase, 2 für NAD(P)H-Oxidase und 3 für HMF-Oxidase.

Die NAD(P)⁺-abhängige Aldehyd-Dehydrogenase umfasst oder besteht bevorzugt aus einer Aminosäuresequenz, welche ausgewählt ist aus der Gruppe bestehend aus:
i) einer Aminosäuresequenz, die eine Identität zu SEQ ID Nr. 2, SEQ ID Nr. 4, SEQ ID Nr. 6, SEQ ID Nr. 8, SEQ ID Nr. 10 oder SEQ ID Nr. 12 von mindestens 80% aufweist,
ii) einer Aminosäuresequenz, die durch eine Nukleinsäure kodiert wird, die eine Identität zu SEQ ID Nr. 1, SEQ ID Nr. 3, SEQ ID Nr. 5, SEQ ID Nr. 7, SEQ ID Nr. 9 oder SEQ ID Nr. 11 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 1, SEQ ID Nr. 3, SEQ ID Nr. 5, SEQ ID Nr. 7, SEQ ID Nr. 9 oder SEQ ID Nr. 11 bindet.

SEQ ID Nr. 1: 
ATGTCACGCTATGAACTGCTGATCGATGGTCGCCTGCAGGCGGCCGAGCACTACGACCGGGTGATCGA
CCCGGCCAGCGAAGAAATCGTTGGCGAAGCCGCCCGCGCCAGCCTGGAGCAGGTCGACGCGGCGGTG
GATGCCGCACACCGCGCCTTCCCGGCCTGGGCCACCGATCTCGACGTCCGCCGCCAGAGCCTGGCCCG
AGCCGCCGAACGAGTACGCGAGAATGCCCAGGCGCTGGCCGAGCTGATCACCCGCGAACAGGGTCGC
CCACTGCGCTCGACCCTGGAGGAAGTGGCTGGCGTCGCCGCCACTTTCGAGCACCACGCGCAGCTGGA
GCTACCCGCCGACACCCAGTTGCGCGACGACGGCGAGCGCCTGGTGCGCATCACCCGCAAACCGCTGG
GGGTGGTCGCCGCGATCACCCCGTGGAACGTCCCGCTGATCCTGCTGGTATTGAAGATCGCGCCTGCC
CTGCACGCCGGCAACACCGTGGTGGCCAAGCCCTCGGAGCACACGCCGCTGTCTACCCTGCTGCTGGC
GCGACTGCTGGGCGATGTGTTCCCCGCTGGCGTGTTCAACGTGGTTGCCGGTGCGGGCGAGGTTGGC
GAACACCTGGTACGTCATCCGCGGGTGCGCCACGTGACCTTCACCGGCAGCGTCGCCACCGGCAAGCG
CCTGTATGCCGGCGCGGGGGACGACCTCAAGCGTCTTACCCTGGAACTGGGCGGCAACGACGCCGCG
CTGGTACTGGAAGACGCCGACCTCGACGCCATCGTCGAACCGCTGTTCTGGGGCGCCTTCTGGAACAG
CGGCCAGGTGTGCTTTGCGATCAAGCGCCTGTACGTGCATGACAGCCTGTTCGAACCGTTACTGGCGA
AACTCGCCGAGCGCGCCCAGCGCACCCGCCTCGGTCATGGGCTCGACCCGCAGACGGAACTGGGGCC
GCTGACCAACGCACAGCAACTGGAACGGGTCATCGCCCTGGTGGAAGACGCCAAGGCTCACGGAGCA
CGCATCCGCAGCGGCGGCGTACGGCCCGACGGTCCCGGCTACTTCTACCCGCCGACCCTGGTCAGCGG
CGTGGCGGCCGGCGTCGCGCTGGTGGACGAGGAACAGTTCGGCCCGGTGTTGCCGGTGATCTCCTTCC
GCAACGAAGAGGACGCCATTACCCAGGCCAACGCCAGCCACTACGGCCTCGGCGCCTCGGTGTGGACC
CGCGACCTGGCGCGCGGCGAAGCCATCGCCAGGCGGCTGGAGGCGGGCCTGGCCTGGGTCAACCAGC
ACGGCCACATCCAGCCCGGCGCGCCCAAGGGCGGGCACAAGTGGAGCGGGCTCGGCTACGAAGGCG
GGCAGCGCGGCTATGAGGCCTTCAGCGAGCTGCAGGTGCTGAACATTTCGCGGCGCTAA
SEQ ID Nr. 2: 
MSRYELLIDGRLQAAEHYDRVIDPASEEIVGEAARASLEQVDAAVDAAHRAFPAWATDLDVRRQSLARAAE
RVRENAQALAELITREQGRPLRSTLEEVAGVAATFEHHAQLELPADTQLRDDGERLVRITRKPLGVVAAITP
WNVPLILLVLKIAPALHAGNTVVAKPSEHTPLSTLLLARLLGDVFPAGVFNVVAGAGEVGEHLVRHPRVRHV
 
TFTGSVATGKRLYAGAGDDLKRLTLELGGNDAALVLEDADLDAIVEPLFWGAFWNSGQVCFAIKRLYVHDS
LFEPLLAKLAERAQRTRLGHGLDPQTELGPLTNAQQLERVIALVEDAKAHGARIRSGGVRPDGPGYFYPPTL
VSGVAAGVALVDEEQFGPVLPVISFRNEEDAITQANASHYGLGASVWTRDLARGEAIARRLEAGLAWVNQ
HGHIQPGAPKGGHKWSGLGYEGGQRGYEAFSELQVLNISRR
SEQ ID Nr. 3: 
ATGTCGACATTTCATTTACTTATTGATGGCCATCTGCAAGCCAGTGACCAATCCGATGTGGTGATCAACC
CCGCGACCGAGCTGGAAGTCGGGCGTGCCCCACGTGCCAGCGCCACCCAGGTCGACCAGGCAGTGGA
AGCCGCACATCAGGCATTTCATCGCTGGGCATCACAGCCTGAGGTGCGCCAGCAGGCACTGCTGGGCG
CGGCCGCCGCCATTCGCCAGCATGCCGATGCCCTGGCACGCCTGATCACGCAAGAGCAGGGGCGGCCA
TTGCACTTTACCCAGGGCGAGGTGGCCGGGGCTGCTGCTACCTTTGAACACTATGCCGGGTTTGCCGCG
CCATCGGATGTGGTGCTGCAGCAGGACGAACAAAAGCGGGTCAGCATTGAGCGCAGGCCGTTTGGCG
TAGTGGCTGCCATTACGCCCTGGAATGTGCCCATCATCCTGCTGGTACTGAAAATCGCCCCGGCCTTGA
AGGCTGGCAATACCGTGGTCGCCAAGCCATCGGAATACACCCCGCTTTCTACCTTGTACCTGGGTGAAA
TTCTGAAAGATGTATTTCCGCCCGGCGTGCTGAACGTGATAGCCGGTGACGGTCAGGTGGGGGCGCGC
CTGGCATCGCATCCGCTGGTGCAGAAGGTGACGTTCACCGGCAGCGTGGCAACCGGAAAAAAACTCTA
TGCCAGCGCCGCGCAGGATGTAAAACGCCTGACGCTGGAACTGGGCGGTAACGATGCGGCCATCGTG
CTGGACGATGCCAATGTCGATGCCATTGCCGAGAAGATTTTCTGGGGCGCCTTCTGGAATAGCGGTCA
GGTGTGCTTTGCCATCAAGCGCCTCTATGTGCACGAGCGTGTTTTCCAGCCCTTGCTCGATGCCCTGGT
GAAACGCGCGCAAAAAACCCGCGTGGGCGATGGCCAGCTGCCGGGTACCGAGCTGGGGCCGCTTACC
AACAAAGCCCAGTTTGAGCGCGTGATATCGCTGGTGGAAGACGCCAGACGCCATGGCGCCACCATTCA
TTCAGGCGGTGCTGCATTGCCTGGCCCCGGCTATTTCTATCCGCCCACCCTGGTCACCGGCATAGGCGC
AGGTGTCGCGCTGGTGGATGAAGAGCAGTTCGGACCAGTATTGCCGCTGATTCCTTTCCGTGATGAGC
AAGAGGCGGTGCGTCAGGCCAACGACAGTCCGTTTGGTCTGGGCGCTTCGGTTTGGACCGCCAATCCC
GAACGCGGCCTGGCGCTGGTACGCCAGCTCCAGGCCGGGCTCGCCTGGGTCAACCAGCATGGCGATAT
CCATCCCGGTGCGCCCAAGGGCGGCTACAAATCCAGTGGCCTTGGCTACGAGGGCGGGCTGCGTGGCT
ATGACGAGTTCAGCGAGCTGCAAGTCGTCAATGCGGCGCTGGTTTAA
SEQ ID Nr. 4: 
MSTFHLLIDGHLQASDQSDVVINPATELEVGRAPRASATQVDQAVEAAHQAFHRWASQPEVRQQALLGA
AAAIRQHADALARLITQEQGRPLHFTQGEVAGAAATFEHYAGFAAPSDVVLQQDEQKRVSIERRPFGVVAA
ITPWNVPIILLVLKIAPALKAGNTVVAKPSEYTPLSTLYLGEILKDVFPPGVLNVIAGDGQVGARLASHPLVQK
VTFTGSVATGKKLYASAAQDVKRLTLELGGNDAAIVLDDANVDAIAEKIFWGAFWNSGQVCFAIKRLYVHE
RVFQPLLDALVKRAQKTRVGDGQLPGTELGPLTNKAQFERVISLVEDARRHGATIHSGGAALPGPGYFYPPT
 
LVTGIGAGVALVDEEQFGPVLPLIPFRDEQEAVRQANDSPFGLGASVWTANPERGLALVRQLQAGLAWVN
QHGDIHPGAPKGGYKSSGLGYEGGLRGYDEFSELQVVNAALV
SEQ ID Nr. 5: 
ATGTCGCGCTATGAACTGCTGATCGATGGTCGCCTCCAGGCGGCCGAGCACTACGACCGGGTTATAGA
CCCGGCCAGCGAAGAACTCGTCGGCGAAGCCGCCCGCGCCAGCCTGGAGCAGGTCGACGCGGCGGTG
GACGCCGCGCACCGCGCCTTCCCAACCTGGGCCACCGATCTCGACGGCCGCCGCCAGAGCCTGGCCCG
AGCCGCCGAACGAGTACGCGAGAACGCCCAGGCGCTGGCCGAGCTGATCACCCGCGAACAGGGTCGC
CCGCTGCGCTCGACACTGGAGGAAGTGGCGGGCGTCGCCGCCACTTTCGAGCACCACGCACATCTGGA
GCTTCCCGCCGATACCCAGTTGCGCGACGACGGCGAACGCCTGGTGCGCATCACCCGCAAACCCCTGG
GGGTGGTCGCCGCAATCACCCCGTGGAACGTCCCGCTGATCCTGCTGGTGCTGAAGATCGCGCCCGCC
CTGCATGCCGGCAACACCGTGGTGGCCAAGCCCTCGGAACACACGCCGCTGTCCACCCTGCTGCTGGC
GCGACTGCTGGGCGATGTGTTCCCTGCTGGCGTGTTCAACGTGATTGCCGGTGCAGGCGAGGTCGGCG
AACACCTGGTGCGCCATCCCCGAGTGCGCCACGTGACCTTCACCGGCAGCGTCGCCACCGGCAAGCGC
CTGTATGCCGGCGCAGCGGACGACCTCAAGCGCCTGACCCTGGAACTGGGCGGCAACGATGCCGCGCT
GGTGCTGGAAGATGCCGACCTCGAAGCCATCGTCGAACCGCTGTTCTGGGGCGCCTTCTGGAACAGCG
GCCAGGTGTGCTTCGCGATCAAGCGCCTGTACGTGCATGAAAGCCTGTTCGAACCGCTGCTGGCCAAA
CTCGCCGAACGCGCCGAGCGCACCCGCCTCGGCCATGGGCTCGACCCGCAGACGGAACTGGGGCCGC
TGACCAACGCGCAGCAACTGGAACGGGTCATCACCCTGGTGGAAGACGCCAAGGCTCACGGAGCGCG
CATCCACAGCGGTGGCACGCGGCCCGACGGCCCCGGTTACTTCTACCCGCCGACCCTGGTCAGCGGCG
TGGCGGCCGGTGTCGCGCTGGTGGATGAGGAACAGTTCGGCCCGGTGCTGCCGGTGATCCCGTTCCGC
GACGAAGAGGACGCCATCGCCCAGGCCAACGCCAGCCCCTACGGCCTCGGCGCCTCGGTGTGGACCC
GCGACCTGGCGCGCGGCGAAGCCATCGCCGAACGGTTGGAGGCGGGCCTGGCCTGGGTCAACCAGCA
CGGCCACATCCAGCCCGGCGCGCCCAAGGGCGGGCACAAGTGGAGCGGGCTCGGCTACGAAGGCGG
GCAACGCGGTTACGAGGCCTTCAGCGAGCTGCAGGTGCTCAACATTTCGCGGCGCTGA
SEQ ID Nr. 6: 
MSRYELLIDGRLQAAEHYDRVIDPASEELVGEAARASLEQVDAAVDAAHRAFPTWATDLDGRRQSLARAAE
RVRENAQALAELITREQGRPLRSTLEEVAGVAATFEHHAHLELPADTQLRDDGERLVRITRKPLGVVAAITP
WNVPLILLVLKIAPALHAGNTVVAKPSEHTPLSTLLLARLLGDVFPAGVFNVIAGAGEVGEHLVRHPRVRHVT
FTGSVATGKRLYAGAADDLKRLTLELGGNDAALVLEDADLEAIVEPLFWGAFWNSGQVCFAIKRLYVHESLF
EPLLAKLAERAERTRLGHGLDPQTELGPLTNAQQLERVITLVEDAKAHGARIHSGGTRPDGPGYFYPPTLVSG
VAAGVALVDEEQFGPVLPVIPFRDEEDAIAQANASPYGLGASVWTRDLARGEAIAERLEAGLAWVNQHGH
IQPGAPKGGHKWSGLGYEGGQRGYEAFSELQVLNISRR
SEQ ID Nr. 7: 
ATGCGTTATGCACATCCTGGCACCCCGGGCGCCTTAGTTTCATTCAAGTCCGCATACGGTAACTATATTG
ACGGTCAATTTGTCGAACCGCTCGGCGGCGAATACTTTATGAATACCTCCCCCGTGGACGGGAGTGAC
ATTGCACAATTTCCCCGCTCCGATGCCAAAGATATCGATTTTGCTCTTGATGCCGCACACCGCGCCGCGG
AAGCCTGGGGGAAAACATCGGTACAGCATCGTTCCAACCTGCTGCTTCAGGTCGCCGATCGGATTCAG
GAGAACCTGGAATATCTGGCGATTGCTGAAAGCTGGGACAATGGTAAACCGGTGCGCGAAACGCTGA
ATGCCGATCTGCCGTTGGCGGTCGATCACTTCCGCTATTTTGCCGGCTGTCTGCGGGCACAGGAGGGG
AGTACTGCGGAAATTGATGAAACCACCGTGGCCTATCACTTCCATGAACCGCTGGGCGTAGTCGGGCA
AATTATTCCGTGGAACTTTCCGCTGCTGATGGCGGCCTGGAAGCTGGCGCCGGCGCTGGCGGCCGGGA
ACTGTGTGGTGCTCAAGCCGGCAGAGCAGACGCCGCTGAGCATCACGCTGCTGCTGGAAATTATTGGC
GACCTCTTCCCGCCGGGCGTCCTGAACGTGGTGCAGGGCTTCGGTAAAGAGGCCGGTGAAGCGCTGG
CAACCAGCAAGCGTATTGCCAAAATTGCCTTTACCGGCTCAACGCCCGTCGGACGGCATATTCTGGCCT
GCGCGGCGGAGAACATTATTCCCGCCACCGTTGAGCTGGGCGGCAAGTCGCCAAATATCTATTTTGCC
GATGTGATGGACGGAGAAGAAGAGTTTATCGAGAAGGCGGTGGAGGGGCTGGTGCTGGGCTTCTTCA
ACCAGGGCGAAGTATGTACCTGCCCATCGCGCGCCCTGATCCATGAATCCATCTACGAGCCGTTTATGG
AGCGAGTGATGGCGAAGGTGGCCCAGATTCGCCGCGGCGATCCCTTTGATACCGACACCATGATCGGG
GCACAGGCATCACGCCAGCAGTTCGATAAGATCCTGTCGTACATTAAGATTGCCCGCGAAGAGGGGGG
GCAGATCCTGACCGGCGGAGAACGCGCCAGTATTACGGCTGAACTGGATAATGGTTACTACATTCAGC
CAACTCTGATTAAGGGGCGCAATGATATGCGCAGCTTCCAGGAAGAGATTTTTGGCCCGGTCATCGGC
GTCACGACCTTCAAAGATGAGGCAGAAGCGCTGGAAATAGCCAACCAGACTCAGTTTGGGCTCGGCGC
CGGCGTCTGGACCCGCGACAGCAATCTGGCGTACCGAATGGGGCGTGGGATTAAAGCCGGTCGTGTCT
GGACCAACTGTTATCACGTCTACCCGGCCCATGCCGCGTTTGGCGGCTATAAACAGTCAGGCGTCGGTC
GTGAAACTCATAAAATGGCCCTTGATGCCTATCAACAGACGAAAAACCTGCTGGTGAGCTACGGTACC
GCGCCGTTAGGACTGTTTTAA
SEQ ID Nr. 8: 
MRYAHPGTPGALVSFKSAYGNYIDGQFVEPLGGEYFMNTSPVDGSDIAQFPRSDAKDIDFALDAAHRAAEA
WGKTSVQHRSNLLLQVADRIQENLEYLAIAESWDNGKPVRETLNADLPLAVDHFRYFAGCLRAQEGSTAEI
DETTVAYHFHEPLGVVGQIIPWNFPLLMAAWKLAPALAAGNCVVLKPAEQTPLSITLLLEIIGDLFPPGVLNV
VQGFGKEAGEALATSKRIAKIAFTGSTPVGRHILACAAENIIPATVELGGKSPNIYFADVMDGEEEFIEKAVEG
LVLGFFNQGEVCTCPSRALIHESIYEPFMERVMAKVAQIRRGDPFDTDTMIGAQASRQQFDKILSYIKIAREE
GGQILTGGERASITAELDNGYYIQPTLIKGRNDMRSFQEEIFGPVIGVTTFKDEAEALEIANQTQFGLGAGVW
TRDSNLAYRMGRGIKAGRVWTNCYHVYPAHAAFGGYKQSGVGRETHKMALDAYQQTKNLLVSYGTAPLG
LF
SEQ ID Nr. 9: 
ATGATCGAACAAAAAATGCTGATCGCCGGCCAGGAATGTGCAGCCGGCAATGGCGCAACGTTTGAGC
GCAAGAATCCGCTGGATGGATCCGTCGCCACACGCGCCCCGGCAGCCACGACCGAGGATGCGATTCGC
GCCTGCGACGCTGCTGCAGCCGCCTTCCCCGCCTGGTCGCAACTGGGGCCGAACGCCCGCCGCGCCAT
GCTGATGAAGGCCTCGCAGGCACTGGAAGCCAAGGGCGAAGCGATTGCTGCGGCCATGGCTGCCGAG
ACCGGCGCCTCGGGCATCTGGGCCGGCTTCAACGTGCATCTGGCCGCCAGCATGCTGCTGGAGGCGGC
TTCGCTGACCACGCAGATCAATGGCGAGATCATTCCCTCCGACGTACCCGGCAGCGTGGCCATGGCCGT
GCGCCAGCCCGCCGGCGTGGTACTCGGTATCGCGCCCTGGAATGCTCCCGTGATCCTGGCCGTGCGCA
GCATCTCCACGGCCCTGGCCTGCGGCAACACCGTGATTCTCAAGGGCTCGGAACTCTGCCCCGCCACCC
ACGGCCTGATCATCGAGGCACTGCAGGACGCAGGCCTGCCCGCCGGCGTGGTGAACTTTGTGACCAAT
GCGCCCGCCGATGCGGGCAGCGTGGTCGAAGCCATCGTGGCCCACCCGGCCGTGCGCCGCGTGAGCT
TTACCGGCTCCACCAAGGTGGGCCGCATCATCGGCCAGACCTGCGCCAGGCACTTGAAGCCTGCGCTG
CTGGAGCTTGGCGGCAAGGCCCCCTTCCTGGTGCTCGACGATGCCGATATCGATGCGGCCGTGAACGC
CGCCACCTTTGGCGCGTTTGCCAACTCGGGCCAGATCTGCATGTCTACCGAGCGCTTTGTGGTGGATAA
CAAGGTGGCCGACGAGTTCATCGCCAAGTTTGCCGCCAAGGCCAGCAGCCTGCCGCTGGGCGACCCGC
GCAAGGGCCCCGTGGTGCTGGGCTCGGTCGTCGATCTGGCTACGGTGGAGCGCTGCAATGCCATGATC
GACGACGCGCTGGCCAAGGGCGCAAAAATCGTCTGCGGCGGCAAGGCCGAGAGCACGCTGATGCCCG
CCACGCTGATCGACCATGTGACGCCGGCGATGCGCATATTCCACGAGGAGACCTTTGGCCCGGTCAAA
GGCATCGTGCGCGTGAACGGCGAGGAAGAAGCCATTGCCACGGCCAATGACAACGAGTTCGGTCTGT
CCTCCGCAGTCTTCACCCGGGACACGGCCCGCGGCTGGCGTGTGGCGGCACGCATCGAGGCCGGCATC
TGCCACATCAACGGCCCCACGGTGCATGACGAAGCCCAGATGCCGTTTGGTGGCGTCAAGGCCTCGGG
CTATGGCCATTTCGGGGGCCAGCAGGGCATCAATGCCTTTACCGAAACCCGCTGGGTGACCATGCAGA
CCGCAGAACGCCACTATCCGTTCTAA
SEQ ID Nr. 10: 
MIEQKMLIAGQECAAGNGATFERKNPLDGSVATRAPAATTEDAIRACDAAAAAFPAWSQLGPNARRAML
MKASQALEAKGEAIAAAMAAETGASGIWAGFNVHLAASMLLEAASLTTQINGEIIPSDVPGSVAMAVRQP
AGVVLGIAPWNAPVILAVRSISTALACGNTVILKGSELCPATHGLIIEALQDAGLPAGVVNFVTNAPADAGSV
VEAIVAHPAVRRVSFTGSTKVGRIIGQTCARHLKPALLELGGKAPFLVLDDADIDAAVNAATFGAFANSGQIC
MSTERFVVDNKVADEFIAKFAAKASSLPLGDPRKGPVVLGSVVDLATVERCNAMIDDALAKGAKIVCGGKA
ESTLMPATLIDHVTPAMRIFHEETFGPVKGIVRVNGEEEAIATANDNEFGLSSAVFTRDTARGWRVAARIEA
GICHINGPTVHDEAQMPFGGVKASGYGHFGGQQGINAFTETRWVTMQTAERHYPF
SEQ ID Nr. 11: 
ATGACTGTCTACGCAAATCCAGGAACCGAAGGCTCGATCGTTAACTATGAAAAGCGCTACGAGAACTA
CATTGGTGGCAAGTGGGTTCCACCGGTAGAGGGCCAGTACCTTGAGAACATTTCACCTGTCACTGGTG
AAGTTTTCTGTGAGGTCGCACGTGGCACCGCAGCGGACGTGGAGCTTGCACTGGATGCTGCACATGCA
GCCGCTGATGCGTGGGGCAAGACTTCTGTCGCTGAACGTGCTCTGATCCTGCACCGCATTGCGGACCG
CATGGAAGAGCACCTGGAAGAAATCGCAGTTGCAGAAACCTGGGAGAACGGCAAGGCAGTCCGTGAG
ACTCTTGCTGCAGATATCCCACTGGCAATCGACCACTTCCGCTACTTTGCTGGCGCGATCCGTGCTCAGG
AAGATCGTTCCTCACAGATCGACCACAACACTGTTGCTTACCACTTCAACGAGCCAATCGGTGTTGTTG
GTCAGATCATTCCTTGGAACTTCCCAATCCTCATGGCTACCTGGAAGCTCGCACCGGCACTTGCTGCAG
GTAACGCGATCGTCATGAAGCCAGCTGAGCAGACCCCAGCATCCATTTTGTATCTGATTAACATCATCG
GCGATCTCATCCCAGAGGGCGTCCTCAACATCGTCAACGGACTCGGCGGTGAAGCAGGCGCTGCACTG
TCCGGCTCTAATCGGATTGGCAAGATTGCTTTCACCGGTTCCACCGAGGTCGGCAAGCTGATCAACCGC
GCTGCATCCGACAAGATCATTCCTGTCACCCTGGAGCTCGGCGGTAAGTCCCCATCCATCTTCTTCTCCG
ATGTTCTGTCACAGGATGACGCCTTCGCAGAGAAGGCAGTTGAAGGCTTCGCGATGTTCGCCCTCAATC
AGGGTGAAGTTTGTACCTGTCCTTCCCGTGCACTTGTTCATGAGTCCATCGCTGATGAATTCCTCGAGCT
TGGCGTGAAGCGAGTTCAGAACATCAAGCTGGGTAACCCACTTGATACTGAAACCATGATGGGTGCTC
AGGCGTCCCAGGAGCAGATGGACAAGATCTCCTCCTACCTGAAGATCGGCCCAGAAGAAGGCGCTCAA
ACCCTCACTGGTGGCAAGGTCAACAAGGTTGATGGCATGGAGAACGGTTACTACATTGAGCCAACCGT
TTTCCGCGGCACCAACGACATGAGGATCTTCCGCGAGGAAATCTTCGGACCAGTCCTTTCTGTTGCTAC
CTTCAGCGACTTCGATGAGGCCATCCGTATTGCAAACGACACCAACTACGGCCTCGGCGCTGGTGTCTG
GAGCCGTGACCAAAACACCATTTATCGTGCAGGTCGCGCAATCCAGGCTGGTCGAGTTTGGGTCAACC
AGTACCACAACTACCCAGCGCACTCCGCTTTCGGTGGATACAAGGAGTCCGGCATCGGCCGTGAGAAC
CACCTCATGATGCTGAACCACTACCAGCAGACCAAGAACCTGTTGGTCTCCTACGATCCAAACCCAACC
GGACTGTTCTAA
SEQ ID Nr. 12:

MTVYANPGTEGSIVNYEKRYENYIGGKWVPPVEGQYLENISPVTGEVFCEVARGTAADVELALDAAHAAAD
AWGKTSVAERALILHRIADRMEEHLEEIAVAETWENGKAVRETLAADIPLAIDHFRYFAGAIRAQEDRSSQID
HNTVAYHFNEPIGVVGQIIPWNFPILMATWKLAPALAAGNAIVMKPAEQTPASILYLINIIGDLIPEGVLNIVN
GLGGEAGAALSGSNRIGKIAFTGSTEVGKLINRAASDKIIPVTLELGGKSPSIFFSDVLSQDDAFAEKAVEGFA
MFALNQGEVCTCPSRALVHESIADEFLELGVKRVQNIKLGNPLDTETMMGAQASQEQMDKISSYLKIGPEE
GAQTLTGGKVNKVDGMENGYYIEPTVFRGTNDMRIFREEIFGPVLSVATFSDFDEAIRIANDTNYGLGAGV
WSRDQNTIYRAGRAIQAGRVWVNQYHNYPAHSAFGGYKESGIGRENHLMMLNHYQQTKNLLVSYDPNP
TGLF

Die hier angeführten NAD(P)⁺-abhängigen Aldehyd-Dehydrogenasen umfassen vorzugsweise eine Aminosäuresequenz, die eine Identität zu SEQ ID Nr. 2, SEQ ID Nr. 4, SEQ ID Nr. 6, SEQ ID Nr. 8, SEQ ID Nr. 10 oder SEQ ID Nr. 12 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist.

Alternativ umfassen die NAD(P)⁺-abhängigen Aldehyd-Dehydrogenasen vorzugsweise eine Aminosäuresequenz, die durch eine Nukleinsäure kodiert wird, die eine Identität zu SEQ ID Nr. 1, SEQ ID Nr. 3, SEQ ID Nr. 5, SEQ ID Nr. 7, SEQ ID Nr. 9 oder SEQ ID Nr. 11 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die Nukleinsäure, welche die erfindungsgemäße NAD(P)⁺-abhängigen Aldehyd-Dehydrogenase kodiert, die Nukleinsäuresequenz SEQ ID Nr. 1, SEQ ID Nr. 3, SEQ ID Nr. 5, SEQ ID Nr. 7, SEQ ID Nr. 9 oder SEQ ID Nr. 11 oder besteht aus dieser.

Der Begriff "Identität", wie hier verwendet, bezieht sich auf den Prozentsatz der identischen Nukleotid- bzw. Aminosäureübereinstimmungen zwischen mindestens zwei, unter Verwendung eines standardisierten Algorithmus miteinander ausgerichteten Nukleotid- bzw. Aminosäuresequenzen ("Alignment"). Ein solcher Algorithmus kann, in einer standardisierten und reproduzierbaren Weise, Lücken ("Gap") in die verglichenen Sequenzen einfügen, um das Alignment zwischen zwei Sequenzen zu optimieren, und somit einen aussagekräftigeren Vergleich der beiden Sequenzen erreichen.

Die prozentuale Identität zwischen Sequenzen kann unter Verwendung von ein oder mehreren Computeralgorithmen oder im Stand der Technik bekannten oder hierin beschriebenen Programmen bestimmt werden. Erfindungsgemäß wird zur Bestimmung der Identität das durch National Center for Biotechnology Information (NCBI) bereitgestellte Basic Local Alignment Search Tool (BLAST) (Altschul et al., 1990) verwendet. Die BLAST-Software-Reihe enthält verschiedene Programme, einschließlich ein als "BLAST 2 Sequences" genanntes Werkzeug, das für den direkten paarweisen Vergleich von zwei Nukleotid- bzw. Aminosäuresequenzen verwendet wird. "BLAST 2 Sequences" kann auch über die NCBI World Wide Web-Seite im Internet interaktiv abgerufen und verwendet werden. Das blastn-Programm (für Nukleotidsequenzen) verwendet als Vorgaben eine Wortlänge (W) von 11, eine Erwartung (E) von 10, M = 5, N = -4 und einen Vergleich beider Stränge. Für Aminosäuresequenzen verwendet das blastp-Programm als Vorgaben eine Wortlänge von 3 und eine Erwartung (E) von 10 und die BLOSUM62-Scoring-Matrix (Henikoff & Henikoff, 1989), Alignments (B) von 50, Erwartung (E) von 10, M = 5, N = -4. Alternativ umfassen die NAD(P)⁺-abhängigen Aldehyd-Dehydrogenasen vorzugsweise eine Aminosäuresequenz, die durch eine Nukleinsäure kodiert wird, die unter stringenten Bedingungen an ein Nukleinsäuremolekül mit der Nukleinsäuresequenz SEQ ID Nr. 1, SEQ ID Nr. 3, SEQ ID Nr. 5, SEQ ID Nr. 7, SEQ ID Nr. 9 oder SEQ ID Nr. 11 bindet. Wie hierin verwendet, beziehen sich die stringenten Bedingungen auf Bedingungen, unter denen sogenannte spezifische Hybride, jedoch keine unspezifischen Hybride gebildet werden. Beispielsweise umfassen die stringenten Bedingungen eine Hybridisierung in 6xSSC (Natriumchlorid/Natriumcitrat) bei 45 °C und dann Waschen mit 0,2 bis 1xSSC, 0,1% SDS bei 50 bis 65 °C; oder derartige Bedingungen können eine Hybridisierung in 1xSSC bei 65 bis 70 °C und dann Waschen mit 0,3xSSC bei 65 bis 70 °C umfassen. Die Hybridisierung kann durch herkömmlich bekannte Verfahren, wie etwa jene, die von J. Sambrook et al. in Molecular Cloning, A Laboratory Manual, 2nd Ed., Cold Spring Harbor Laboratory (1989), beschrieben sind, durchgeführt werden.

Offenbart ist ferner die Verwendung einer NAD(P)⁺-abhängigen Aldehyd-Dehydrogenase, wobei die NAD(P)⁺-abhängige Aldehyd-Dehydrogenase eine Aminosäuresequenz umfasst, welche ausgewählt ist aus der Gruppe bestehend aus:
i) einer Aminosäuresequenz, die eine Identität zu SEQ ID Nr. 2, SEQ ID Nr. 4, SEQ ID Nr. 6, SEQ ID Nr. 8, SEQ ID Nr. 10 oder SEQ ID Nr. 12 von mindestens 80% aufweist,
ii) einer Aminosäuresequenz, die durch eine Nukleinsäure kodiert wird, die eine Identität zu SEQ ID Nr. 1, SEQ ID Nr. 3, SEQ ID Nr. 5, SEQ ID Nr. 7, SEQ ID Nr. 9 oder SEQ ID Nr. 11 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 1, SEQ ID Nr. 3, SEQ ID Nr. 5, SEQ ID Nr. 7, SEQ ID Nr. 9 oder SEQ ID Nr. 11 bindet.

In der bevorzugten Variante des erfindungsgemäßen Verfahrens wird die enzymatische Oxidation von NAD(P)H zu NAD(P)⁺ mittels einer NAD(P)H-Oxidase bewerkstelligt.

Die zur Kofaktor-Regeneration eingesetzte NAD(P)H-Oxidase kann aus einer der Gruppen EC 1.6.3.1 (NAD(P)H-Oxidase (H₂O₂-bildend)), EC 1.6.3.2 (NAD(P)H-Oxidase (H₂O-bildend)), EC 1.6.3.3 (NADH-Oxidase (H₂O₂-bildend)) sowie EC 1.6.3.4 (NADH-Oxidase (H₂O-bildend)) stammen, wobei die H₂O-bildenden Klassen besonders bevorzugt sind.

Eine besonders bevorzugt eingesetzte H₂O-bildende NAD(P)H-Oxidase umfasst oder besteht vorzugsweise aus eine Aminosäuresequenz, die ausgewählt ist aus der Gruppe bestehend aus:
i) einer Aminosäuresequenz, die eine Identität zu SEQ ID Nr. 14, SEQ ID Nr. 16 oder SEQ ID Nr. 18 von mindestens 80% aufweist,
ii) einer Aminosäuresequenz, die durch eine Nukleinsäure kodiert wird, die eine Identität zu SEQ ID Nr. 13, SEQ ID Nr. 15 oder SEQ ID Nr. 17 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 13, SEQ ID Nr. 15 oder SEQ ID Nr. 17 bindet.

SEQ ID Nr. 13: 
ATGAAAGTAGTAGTAGTAGGCTGTACACATGCAGGAACAGCGGCAGTTAAGACGATTTTAAATGAACA
TCCAGATGCATCAGTATCAGTATATGAGCGTAATGACAATGTCTCATTTCTATCTTGTGGGATTGCGTTG
TATGTTGGTGGAGTTGTGAAAGATCCTGCAGGTTTGTTTTATTCAAGTCCAGAAGAACTTGCATCAATG
GGCGCGAAAATTAACATGGAACACAATGTGAAAAATATAGATAATGAGAATAAGGTCGTAGTAATTGA
GAATTTAAAAACAGGCGAAACATTTGAAGAAAGCTATGATAAGTTGGTAATGACAACTGGATCATGGC
CAATTATTCCTCCAATTGATGGAATCAATAGTGAAAATATTCTTTTGTGTAAAAACTATAACCAAGCAAA
TGAAATTATTAAAGAATCAAAAAATGCTAAAAAGATTGTCATTGTTGGTGGTGGCTATATTGGAATTGA
ATTAGTTGAGGCATTTGCAGAATCTGGCAAGCAAGTGACGCTAGTTGATGGATTAGATCGTATTTTAAA
CAAATATTTAGATGCTGAATTCACTTCTGTTTTAGAGCATGATTTACAAGAAAGAGGCGTTACGCTAGCT
TTAAACCAAACCGTCGAGAAATTTGTTGCCAATGAATCAGGTGCTGTGACAGCTGTGAAAACACCAGTT
GGAGAATATGAGGCTGATTTAGTTATTTTATGTGTTGGATTTAAACCAAATACTGATTTGTTGAAGGAT
AAAGTAGAGATGTTGCCAAATGGTGCCATCGTAGTGGATGAATATATGAGAACAAGCGATGAAGCGAT
TTTTGCTGCTGGCGATAGTTGCGCGGTTCATTATAATCCAACTGGAGGCTCTGCGTATATTCCGTTAGCT
ACAAATGCAGTTAGAATGGGAGCTTTAGTTGGGAAAAATATTGTTTCTCCAACAGTTAAATATCGTGGC
ACGCAAGCAACTTCTGGTTTATATTTATTTGGTTTTAATATAGGTTCAACCGGATTGACTGAAAATAGCG
CTCCTCATTTTGGCGTAGAGGTTCGTTCAGTAGTTGTAGAAGATAATTATCGTCCAGAGTTTATGCCGAC
AACAGAGAAAGTAACGATGAAATTAGTTTATGAAGTAGGAACGAATCGGATTGTTGGAGGTCAAATCA
TGTCAAAATATGATGTGACACAATCTGCCAATACGTTATCTTTATGTGTTCAAAATAAAATGACGATTGA
GGATTTGGCTTATGTAGATTTCTTCTTCCAACCTCACTTTGATCGTCCTTGGAACTATTTAAATATTTTAG
CGCAAGCAGCTGTTGAGCAAGAGCGTAAACTAGCAAAATAA
SEQ ID Nr. 14: 
MKVVVVGCTHAGTAAVKTILNEHPDASVSVYERNDNVSFLSCGIALYVGGVVKDPAGLFYSSPEELASMGA
KINMEHNVKNIDNENKVVVIENLKTGETFEESYDKLVMTTGSWPIIPPIDGINSENILLCKNYNQANEIIKESK
NAKKIVIVGGGYIGIELVEAFAESGKQVTLVDGLDRILNKYLDAEFTSVLEHDLQERGVTLALNQTVEKFVANE
 
SGAVTAVKTPVGEYEADLVILCVGFKPNTDLLKDKVEMLPNGAIVVDEYMRTSDEAIFAAGDSCAVHYNPT
GGSAYIPLATNAVRMGALVGKNIVSPTVKYRGTQATSGLYLFGFNIGSTGLTENSAPHFGVEVRSVVVEDNY
RPEFMPTTEKVTMKLVYEVGTNRIVGGQIMSKYDVTQSANTLSLCVQNKMTIEDLAYVDFFFQPHFDRPW
NYLNILAQAAVEQERKLAK
SEQ ID Nr. 15: 
ATGAAAGTTGCAGTAATCGGTTGTACCCATGCTGGGACAGCTGCCGTTAAAACTATCTTAACAGAAAAC
GATGATGTAGAAGTTGTTGTTTTTGAACGCAATGATAATATCTCCTTCTTATCTTGTGGGATTGCCCTTT
ACGTAGGTGGCGTGGTCAAAGACGTCAATGGCCTTTTCTATTCTGATCCAAGTGAGTTAGAATCTCTAG
GCGCAACAGTTTATATGAAACACAATGTGCTATCGTTTGACGAAAACACTAAAGTTATCCAAGTAGAAA
ATATGGAGACTGGCGAACATTTCCAAGAATCATATGACAAGTTAGTCATTGCCACTGGTTCTTGGCCGA
TTATTCCTGATTTACCAGGACTAGATCTAGAAAATGTCATGCTATGTAAAAACTTTAAACATGCCCAAGA
ATTAATCCAAACTAAGCAAGATAAAAAACGAGTTGCTGTTATTGGTGCTGGTTATATTGGTATCGAGTT
AGTTGAAGCCTTTGCTGAAGACGGTAAAGAAGTTGTTTTAATCGATGGTGCTGACCGCGTCCTACCTAA
GTATCTCGACCAGGAAATGACTGACTTATTAGAAGCTAGCTTGGTAGACCATGGCGTACAAATGCAATT
AGGGGAATTTGTAGAATCATTCCTAGCCGACGATGAAGGTAAGGTACGTGCCGTTAAAACGTCTAAAG
GTGAATATGAATGTGATATGGCAGTCCTTTGTGTCGGCTTCCAACCTAACACAGAATTATATAAGGGCA
AGTTAGAAACTATGCCGAATGGTGCGATTATTGTAGATGACTATATGCACACCTCTCACCCAGATATCT
ATGCCTGTGGTGACTCATGTGCTGTCAACTATAATCCTAACGATGGCCATGCTTATATCCCGCTTGCCAC
TAATGCTGTCCGTATGGGTAGCTTAGTTGGTAAAAATATTAAAGCAGACCGCGTCAAATACCGGGGCA
CCCAGTCAACATCTGGTCTAAAATTATTCGGCTGGAATATTGGCTCAACTGGTGTTACCGACAATTCTGC
TAGCAGCTTCAACCTTGAAACTCGTAGCGTCTATGTTGAAGATAACTACCGCCCTGAATTCATGCCTACT
ACAGAAAAAGTTTATATGAAGTTAGTCTATGAAGTAGGCACAAATCGAGTTGTTGGCGGTCAGCTTAT
GTCTAAGTATGATATTACCCAATCAGCCAACACCCTATCACTAGCTATCCAAACCAAGCAAACAATTGAA
GATTTAGCCTATGTGGACTTCTTCTTCCAACCACACTTTGACCGCCCTTGGAACTATTTAAATATTTTAGC
CCAAGCAGCACTAAGTCAAGAAGAAGAATTAGCTCAAAACTAA
SEQ ID Nr. 16: 
MKVAVIGCTHAGTAAVKTILTENDDVEVVVFERNDNISFLSCGIALYVGGVVKDVNGLFYSDPSELESLGATV
YMKHNVLSFDENTKVIQVENMETGEHFQESYDKLVIATGSWPIIPDLPGLDLENVMLCKNFKHAQELIQTK
QDKKRVAVIGAGYIGIELVEAFAEDGKEVVLIDGADRVLPKYLDQEMTDLLEASLVDHGVQMQLGEFVESFL
ADDEGKVRAVKTSKGEYECDMAVLCVGFQPNTELYKGKLETMPNGAIIVDDYMHTSHPDIYACGDSCAVN
YNPNDGHAYIPLATNAVRMGSLVGKNIKADRVKYRGTQSTSGLKLFGWNIGSTGVTDNSASSFNLETRSVY
 
VEDNYRPEFMPTTEKVYMKLVYEVGTNRVVGGQLMSKYDITQSANTLSLAIQTKQTIEDLAYVDFFFQPHF
DRPWNYLNILAQAALSQEEELAQN
SEQ ID Nr. 17: 
ATGAAAGTAGTCGTTGTAGGATGTACGCACGCTGGAACATCAGCAGTAAAAACAATTCTTAATGAGCA
TCCAAATACTGAAGTAACAGTTTTTGAACGAAATGATAATGTGTCATTCTTATCATGCGGAATCGCATTG
TATGTTGGTGGCGTTGTGAAAGATCCAGCAGGTTTATTCTATTCAAATCCTGAAGAATTAACTGAAATG
GGCGCAACTGTGCACATGGAACACAATGTTACAAATATCGATACTGTTGCTAAAAAAGTTACTGTAGAA
AATATGCAAACTGGTGAAGTCTTTGAAGAATCATACGACAAATTAGTGAATACAACAGGTTCATGGCCG
ATTGTTCCACCAATCTCTGGTATTGAGTCTAAAAACATTTTATTATGTAAAAACTACAACCAAGCTAATG
AAATCATTCGCCAAGCAAAAGACAAACAAAAAGTTGTTATTGTTGGTGGAGGTTACATTGGTATTGAAT
TAGTGGAAGCGTTTGCTGAATCTGGAAAAGATGTTACGTTGATTGATGGATTAGACCGTATTTTAAACA
AATACCTAGATCCAGAATTCACAGATATCTTAGAACACGATTTGCAAGAACGTGGAATCAAATTAGCAC
TAAACCAAACTGTAAATGGCTTTGAAGCGAATGAAAATGGAGAAGTAACTAAAGTTGTCACTTCTGAA
AATTCATTTGAAACTGAAATGGTTATCATGTGTGTTGGTTTCCGTCCAAACAATGAATTGTTAAAAGACA
AAGTAGATATGTTGCCAAATGGCGCTATTATTGTTGATGAATACATGAGAACAAGCGATAAAGATATTT
ACGCTGCTGGAGACAGCTGTGCAGTTCATTACAATCCAAATGGTGGATCTGCATATATTCCATTAGCAA
CTAACGCTGTTCGCATGGGAACTTTAGTTGGTAAAAACATTGTTGAACCAAGTGTTAAATACCGTGGAA
CACAATCTACATCTGGATTGTACTTGTTCGGTTTCAACATCGGTTCTACAGGAGTAAACGTAAACAGTGC
TTCTCATTTCGGATTAGATGTTCGTTCAGTTGTAGTTGAAGACTACTACCGTCCAGAATTCATGCCGACA
AATGAAAAAGTATTGATGAAATTAGTTTATGAAGTTGGAACAAACCGCATCGTTGGTGGACAAGTAAT
GTCTAAATATGACATCACTCAATCAGCTAATACATTGTCATTAGCTGTACAAAACAAAATGACTATCGAA
GATTTGGCATATGTTGACTTCTTCTTCCAACCAGTATTCGATCGTCCTTGGAACTACTTGAACTTGCTTGC
ACAAGCAGCAGTAGAACAAGAAAGAAAAATCGCAACAGGTACAGAAGTAACTGTTTAA
SEQ ID Nr. 18:

MKVVVVGCTHAGTSAVKTILNEHPNTEVTVFERNDNVSFLSCGIALYVGGVVKDPAGLFYSNPEELTEMGA
TVHMEHNVTNIDTVAKKVTVENMQTGEVFEESYDKLVNTTGSWPIVPPISGIESKNILLCKNYNQANEIIRQ
AKDKQKVVIVGGGYIGIELVEAFAESGKDVTLIDGLDRILNKYLDPEFTDILEHDLQERGIKLALNQTVNGFEA
NENGEVTKVVTSENSFETEMVIMCVGFRPNNELLKDKVDMLPNGAIIVDEYMRTSDKDIYAAGDSCAVHY
NPNGGSAYIPLATNAVRMGTLVGKNIVEPSVKYRGTQSTSGLYLFGFNIGSTGVNVNSASHFGLDVRSVVVE
DYYRPEFMPTNEKVLMKLVYEVGTNRIVGGQVMSKYDITQSANTLSLAVQNKMTIEDLAYVDFFFQPVFDR
PWNYLNLLAQAAVEQERKIATGTEVTV

Die bevorzugt verwendete H₂O-bildende NAD(P)H-Oxidase umfasst oder besteht vorzugsweise aus einer Aminosäuresequenz, die eine Identität zu SEQ ID Nr. 14, SEQ ID Nr. 16 oder SEQ ID Nr. 18 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die H₂O-bildende NAD(P)H-Oxidase die Aminosäuresequenz SEQ ID Nr. 14, SEQ ID Nr. 16 oder SEQ ID Nr. 18 oder besteht aus dieser.

Alternativ umfasst die H₂O-bildende NAD(P)H-Oxidase vorzugsweise eine Aminosäuresequenz, die durch eine Nukleinsäure kodiert wird, die eine Identität zu SEQ ID Nr. 13, SEQ ID Nr. 15 oder SEQ ID Nr. 17 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die Nukleinsäure, welche die H₂O-bildende NAD(P)H-Oxidase kodiert, die Nukleinsäuresequenz SEQ ID Nr. 13, SEQ ID Nr. 15 oder SEQ ID Nr. 17 oder besteht aus dieser.

Alternativ umfasst die H₂O-bildende NAD(P)H-Oxidase vorzugsweise eine Aminosäuresequenz, die durch eine Nukleinsäure kodiert wird, die unter stringenten Bedingungen an ein Nukleinsäuremolekül mit der Nukleinsäuresequenz SEQ ID Nr. 13, SEQ ID Nr. 15 oder SEQ ID Nr. 17 bindet. Wie hierin verwendet, beziehen sich die stringenten Bedingungen auf Bedingungen, unter denen sogenannte spezifische Hybride, jedoch keine unspezifischen Hybride gebildet werden. Beispielsweise umfassen die stringenten Bedingungen eine Hybridisierung in 6xSSC (Natriumchlorid/Natriumcitrat) bei 45 °C und dann Waschen mit 0,2 bis 1xSSC, 0,1% SDS bei 50 bis 65 °C; oder derartige Bedingungen können eine Hybridisierung in 1xSSC bei 65 bis 70 °C und dann Waschen mit 0,3xSSC bei 65 bis 70 °C umfassen. Die Hybridisierung kann durch herkömmlich bekannte Verfahren, wie etwa jene, die von J. Sambrook et al. in Molecular Cloning, A Laboratory Manual, 2nd Ed., Cold Spring Harbor Laboratory (1989), beschrieben sind, durchgeführt werden.

Ein weiterer Aspekt der vorliegenden Erfindung betrifft die Verwendung einer H₂O-bildenden NAD(P)H-Oxidase, welche eine Aminosäuresequenz umfasst oder daraus besteht, die ausgewählt ist aus der Gruppe bestehend aus:
i) einer Aminosäuresequenz, die eine Identität zu SEQ ID Nr. 14, SEQ ID Nr. 16 oder SEQ ID Nr. 18 von mindestens 80% aufweist,
ii) einer Aminosäuresequenz, die durch eine Nukleinsäure kodiert wird, die eine Identität zu SEQ ID Nr. 13, SEQ ID Nr. 15 oder SEQ ID Nr. 17 von mindestens 80% aufweist, und
iii) einer Aminosäuresequenz, die durch eine Nukleinsäure kodiert wird, die unter stringenten Bedingungen an ein Nukleinsäuremolekül mit der SEQ ID Nr. 13, SEQ ID Nr. 15 oder SEQ ID Nr. 17 bindet.

In weiteren bevorzugten Ausführungsform des erfindungsgemäßen Verfahrens beträgt die Konzentration von HMF in der wässerigen Lösung 15 - 130 g/l.

Der besonders bevorzugte Temperaturbereich liegt zwischen 18 und 40 °C.

Der besonders bevorzugte pH-Bereich der Reaktion liegt zwischen pH 6 und pH 9.

Bei einer weiteren, bevorzugten Variante des erfindungsgemäßen Verfahrens liegen die Enzyme in einer Suspension, im Homogenat und/oder im Lysat der entsprechenden, sie bildenden Zellen vor, wobei Lysate besonders bevorzugt sind.

Suspension bedeutet in diesem Kontext eine Suspension von *resting cells.* Diese werden nach der Kultivierung geerntet (vom Nährmedium abgetrennt) und in einem geeigneten Puffersystem suspendiert. Im Gegensatz zu fermentativen Verfahren, bei denen auch mit ganzen Zellen gearbeitet wird, können die *resting cells* aufgrund der Entfernung von Kohlenstoff-Quellen und Nährstoffen nicht mehr wachsen, sondern dienen nur der Umsetzung von Substraten (Lin & Tao, 2017). Homogenat steht in diesem Kontext für eine physikalisch und/oder chemisch behandelte Suspension (z.B. mittels Druckes, Lysozym oder Ultraschall behandelt), wobei die Zellbestandteile aus den Zellen freigesetzt werden. Ein Lysat wird erhalten, wenn die unlöslichen Zellbestandteile des Homogenats beispielsweise durch Filtration oder Zentrifugation entfernt werden (siehe *Produktion der Enzyme* & *Herstellung der Lysate* für Details).

In einer weiteren Variante können die Enzyme auch mit einem wasserlöslichen Polymer wie Polyethylenglycol am N-Terminus modifiziert, in oder auf einer festen Matrix immobilisiert oder Teil eines Fusionsproteins sein.

In einer weiteren Variante können die Enzyme in Pulverform, in lyophilisierter oder sprühgetrockneter Form vorliegen.

Mit den nachfolgenden Beispielen werden bevorzugte Ausführungsformen der Erfindung noch näher beschrieben.

### Materialien

5-(Hydroxymethyl)furfural (HMF) wurde von Biosynth, 5-Formyl-2-furancarbonsäure (FFA) sowie 2,5-Furandicarbonsäure (FDCA) wurden von TCI, 2,5-Diformylfuran (DFF) und 5-Hydroxymethyl-2-furancarbonsäure (HMFA) wurden von Sigma-Aldrich, Aceton, Kaliumdihydrogenphosphat, di-Kaliumhydrogenphosphat sowie Natriumdodecylsulfat (SDS) wurden von Carl Roth, NAD⁺, NADH-Dinatriumsalz, NADP⁺-Dinatriumsalz, NADPH-Tetranatriumsalz sowie Acetonitril wurden von PanReac AppliChem (ITW Reagents) und Triethanolamin wurde von Chem-Lab NV bezogen. Die kommerzielle NADH-Oxidase NOX-009 wurde von Prozomix Limited, UK bezogen.

### Produktion der Enzyme & Herstellung der Lysate

### Allgemeines zur Expression von rekombinanten Enzymen in E. coli

Für die rekombinante Enzymproduktion in einem *Escherichia* coli-Stamm wurde zunächst das zu exprimierende Gen in einer PCR unter der Verwendung der genomischen DNA oder deren synthetisch an die Codon-Verwendung von *E. coli* angepasstes Äquivalent als Matrize zusammen mit spezifischen Oligonukleotiden, die zusätzlich Erkennungssequenzen für Restriktionsendonukleasen tragen, amplifiziert und aus dem Reaktionsgemisch isoliert. Nach dem Nukleinsäure-Verdau mit den Restriktionsenzymen Sphl und Hindlll wurde das für das Target-Enzym kodierende Genfragment in das mit Sphl und Hindlll geschnittene Rückgrat des Expressionsvektors pQE70-Kan ligiert. Das Ligationsprodukt wurde in chemisch kompetente *E*. *coli*-Zellen Top10F' transformiert und die entstandenen Kolonien wurden für die Plasmid-Isolierung und Restriktionsanalyse benutzt.

Das Ergebnis des Klonierungs-Schritts wurde mittels Restriktionsenzym-Verdau und DNA-Sequenzierung überprüft. Das resultierende Konstrukt trägt das Target-Gen unter dem IPTG-induzierbaren T5-Promotor.

Für die Überexpression des Enzymes in *E. coli* wurde das resultierende Expressionsplasmid in die kompetenten Expressionszellen RB791 transformiert. Nach 24 h Inkubation bei 37 °C wurden entstandene Kolonien für die Expressionstests in LB-Medium angeimpft.

Am nächsten Tag wurden damit Expressionskulturen mit einer optischen Dichte OD₅₅₀ von 0,02 angeimpft und bei 37 °C geschüttelt, bis eine OD₅₅₀ von 0,3 erreicht wurde. Anschließend wurde die Temperatur auf 25 °C gesenkt und die Kulturen wurden beim Erreichen einer OD₅₅₀ von 0,5 mit 0,1 mM IPTG induziert. Nach 22 h wurden die Kulturen geerntet (vom Medium mittels Zentrifugation in Form eines Zellpellets abgetrennt) und auf die Expression des rekombinanten Enzymes mit Hilfe von SDS-Gelelektrophorese und einer Aktivitätsbestimmung (Verwendung in Use-Test oder optisch-enzymatischer Assay) analysiert.

### Herstellung von Zell-Lysaten mittels Sonifier-Aufschlusses

Zur Herstellung einer Zell-Suspension wurde das nach obigen Verfahren hergestellte Zellpellet in einem geeigneten Gefäß eingewogen und mit Puffer und Lysozym (finale Konzentration 0,5 mg/ml) versetzt (z.B. Kaliumphosphat-Puffer) und unter Rührung gelöst. Der Massenanteil an Biomasse beträgt üblicherweise 20%, der Rest entfällt auf den Puffer.

Zum Zell-Aufschluss wurde ein Branson Sonifier 450 verwendet. Die Suspension wurde dreimal mit je 15 Ultraschall-Stößen (Einstellungen am Gerät: Timer = 15; Duty Cycle = 50; Output Control = 3 - 5) behandelt.

Das erhaltene Homogenat wurde 10 min lang bei 4 °C und 16000 rpm zentrifugiert (Eppendorf Zentrifuge 5417R), um die unlöslichen Zellfragmente abzutrennen und das Lysat zu erhalten.

**Tabelle 1. Enzymtypen und Spenderorganismen für die in den Beispielen verwendeten Enzyme (ALDH = Aldehyd-Dehydrogenase).**

| **Enzymtyp (EC-Klasse)** | **katalysierte Reaktion** | **Spenderorganismus** | **Literatur** |
|---|---|---|---|
| ALDH I | HMF → HMFA; FFA → FDCA | *Pseudomonas nitroreducens* | (NCBI Protein Database: WP_024766379.1); SEQ ID Nr. 2 |
| ALDH II | HMF → HMFA; FFA → FDCA | *Methylovorus glucosotrophus* | (NCBI Protein Database: WP_015829138.1); SEQ ID Nr. 4 |
| ALDH III | HMF → HMFA; FFA → FDCA | *Pseudomonas multiresinivorans* | (NCBI Protein Database: WP_169935101.1); SEQ ID Nr. 6 |
| ALDH IV | HMF → HMFA; FFA → FDCA | *Raoultella ornithinolytica* | (NCBI Protein Database: WP_004866732.1); SEQ ID Nr. 8 |
| ALDH V | HMF → HMFA; FFA → FDCA | *Comamonas testosteroni* | (NCBI Protein Database: WP_003076354.1); SEQ ID Nr. 10 |
| ALDH VI | HMF → HMFA; FFA → FDCA | *Corynebacterium glutamicum* | (NCBI Protein Database: WP_011015386.1); SEQ ID Nr. 12 |
| HMF-Oxidase* (HMFO) | HMF → DFF; DFF → FFA; HMFA → FFA | *Pseudomonas nitroreducens* | (NCBI Protein Database: WP_024766380.1) |
| Alkoholdehydrogenase (ADH; EC 1.1.1.1) | Aceton → 2-Propanol | *(Geo-)Bacillus stearothermophilus* NCA1503 | (Sakoda & Imanaka, 1992) |
| NADH-Oxidase I (EC 1.6.3.4) | NADH → NAD⁺ | *Carnobacterium divergens* | SEQ ID Nr. 14 |
| NADH-Oxidase II (EC 1.6.3.4) | NADH → NAD⁺ | *Aerococcus urinaehominis* | (NCBI Protein Database: WP_067978261.1); SEQ ID Nr. 16 |
| NADH-Oxidase III (EC 1.6.3.4) | NADH → NAD⁺ | *Desemzia incerta* | (NCBI Protein Database: WP_092479712.1); SEQ ID Nr. 18 |
| Katalase | H₂O₂ → H₂O + ½ O₂ | *Micrococcus luteus* | (UniProt: P29422) |

| | | | |
|---|---|---|---|
| * Anmerkung: In der NCBI Protein Database wird die HMF-Oxidase aus *P. nitroreducens* als Glucose-Methanol-Cholin (GMC) Oxidoreduktase klassifiziert, welche als Überfamilie auch die HMF-Oxidasen beinhaltet (Viñambres et al., 2020). | | | |

### Analytische Methoden

### High Performance Liquid Chromatography (HPLC)

Zur Quantifizierung von HMF, DFF, HMFA, FFA und FDCA wurde HPLC (High Performance Liquid Chromatography) verwendet. Die Detektion erfolgte mittels eines UV-Detektors. Zur Messung wurde eine Phenomenex Rezex ROA-Organic Acid H+ (8%) Säule mit entsprechender Vorsäule verwendet und mit 1 mM Schwefelsäure isokratisch eluiert.

### Bestimmung von Enzym-Aktivitäten (optisch-enzymatischer Assay)

Enzym-Aktivitäten in den Lysaten wurden mit einem Shimadzu UV-1900 Spektrophotometer bestimmt. Dazu wurde die Bildung oder der Verbrauch von NAD(P)H bei einer Wellenlänge von 340 nm über die Änderung der Absorption verfolgt. Die Messungen wurden mit 0,2 mM Kofaktor (NAD(P)⁺ oder NAD(P)H) durchgeführt. Dazu wurden 20 µl einer 10 mM Stock-Lösung des Kofaktors in einer Küvette (Greiner bioone Semi-Micro-Küvette aus Polystyrol) vorgelegt und der gewünschte pH-Wert wurde mit 100 mM TEA-HCl-Puffer eingestellt (870 µl). 10 µl Lysat (verdünnt oder unverdünnt) und 100 µl Substrat-Lösung wurden zur Küvette zugesetzt und die Messung unmittelbar danach gestartet. Die Messungen wurden standardmäßig bei 25 °C durchgeführt. Über den Extinktionskoeffizienten von NADH/NADPH bei 340 nm (*ε* = 6220 L mol⁻¹ cm⁻¹) kann die Enzym-Aktivität des Lysats in U/ml (bezogen auf das Volumen des Lysats) oder U/g (bezogen auf die zur Herstellung eingesetzte Biomasse) bestimmt werden. 1 U steht dabei für 1 µmol Substratumsatz pro Minute (1 U = 1 µmol/min = 1,67·10⁻⁸ kat).

Mit den nachfolgenden Beispielen werden bevorzugte Varianten des erfindungsgemäßen Verfahrens noch näher beschrieben. Die in diesen Beispielen eingesetzten Lysate wurden nach den oben beschriebenen Verfahren hergestellt.

### Beispiel 1

### Oxidation von 5-Hydroxymethylfurfural zu 5-Hydroxymethyl-2-furancarbonsäure mit verschiedenen Aldehyd-Dehydrogenasen

Die Oxidationsreaktionen wurden in 2 ml Glas-Vials durchgeführt, die Lösungen von je 30 µl NADH-Oxidase-Lysat sowie 50 µl Aldehyd-Dehydrogenase-Lysat (siehe nachfolgende Tabelle 2) in 250 mM Kaliumphosphat-Puffer (pH 7) enthielten.

Zum Start der Reaktion wurde HMF oder HMFA (finale Konzentration 10 mM) zu den Vials zugegeben. Die Vials wurden in einem Eppendorf-Thermomixer unter kontinuierlichem Schütteln (30 °C, 800 rpm) für 20 h inkubiert.

Zur Analyse wurden 50 µl eines Ansatzes mit 200 µl Acetonitril versetzt und im Eppendorf-Thermomixer bei 85 °C und 1200 rpm für 15 min inkubiert. Die Probe wurde in einer Zentrifuge kurz zentrifugiert, mit 750 µl Reinstwasser versetzt, gevortext und anschließend für 5 min bei max. g zentrifugiert. 200 µl des Überstands wurden in ein HPLC-Vial überführt und mittels HPLC (UV-Detektion) vermessen. Die Ergebnisse sind der nachfolgenden Tabelle 2 dargestellt.

**Tabelle 2**

| **Enzym** | **Umsatz HMF → HMFA [%]** | **Umsatz HMFA → FFA/FDCA [%]** |
|---|---|---|
| ALDH I | 100 | 0 |
| ALDH II | 100 | 0 |
| ALDH III | 100 | 0 |
| ALDH IV | 100 | 0 |
| ALDH V | 100 | 0 |
| ALDH VI | 72 | 0 |

Die Ergebnisse in Tabelle 2 zeigen, dass die hier getesteten Aldehyd-Dehydrogenasen in Kombination mit einer NADH-Oxidase zur Kofaktor-Regeneration nur HMF zu HMFA oxidieren, HMFA wird hingegen nicht weiter oxidiert.

### Beispiel 2

### Oxidation von 5-Hydroxymethylfurfural zu 5-Hydroxymethyl-2-furancarbonsäure - Vergleich verschiedener NADH-Oxidasen

Die Oxidationsreaktionen wurden in 2 ml Glas-Vials durchgeführt, die Lösungen von je 10 µl ALDH I-Lysat, 5 U NADH-Oxidase-Lysat (siehe nachfolgende Tabelle 3) sowie 0,1 mM NAD⁺ in 250 mM Kaliumphosphat-Puffer (pH 7) enthielten.

Zum Start der Reaktion wurde HMF (finale Konzentration 20 g/l) zu den Vials zugegeben. Die Vials wurden in einem Eppendorf-Thermomixer unter kontinuierlichem Schütteln (35 °C, 800 rpm) für 20 h inkubiert.

Zur Analyse wurden 50 µl eines Ansatzes mit 200 µl Acetonitril versetzt und im Eppendorf-Thermomixer bei 85 °C und 1200 rpm für 15 min inkubiert. Die Probe wurde in einer Zentrifuge kurz zentrifugiert, mit 750 µl Reinstwasser versetzt, gevortext und anschließend für 5 min bei max. g zentrifugiert. 300 µl des Überstands wurden in ein HPLC-Vial überführt, mit 300 µl 20% Acetonitril verdünnt und mittels HPLC (UV-Detektion) vermessen. Die Ergebnisse sind der nachfolgenden Tabelle 3 dargestellt.

**Tabelle 3**

| **NADH-Oxidase** | **Ausbeute HMFA [%]** |
|---|---|
| *Carnobacterium divergens* (SEQ ID Nr. 14) | 65 |
| *Aerococcus urinaehominis* (SEQ ID Nr. 16) | 82 |
| *Desemzia incerta* (SEQ ID Nr. 18) | 80 |
| *Streptococcus mutans* | 42 |

Die Tabelle 3 zeigt, dass die erfindungsgemäß verwendeten NADH-Oxidasen (SEQ ID Nr. 14, SEQ ID Nr. 16, SEQ ID Nr. 18) im Vergleich zu der von Knaus et al. (2018) beschriebenen NADH-Oxidase aus *Streptococcus mutans* zu wesentlich höheren HMFA-Ausbeuten führen.

### Beispiel 3

### Oxidation von 5-Hydroxymethylfurfural zu 5-Hydroxymethyl-2-furancarbonsäure - Vergleich verschiedener NADH-Oxidasen

Die Oxidationsreaktion wurde in zweifacher Ausführung in je einem Labfors Tisch-Bioreaktor (Infors AG) durchgeführt. Als Gefäß wurde ein Glasreaktor (Volumen 3,4 l) mit aufgesetztem Rührwerk, O₂-Sensor und pH-Elektrode verwendet. Die pH-Kontrolle erfolgte durch die Zudosierung von 5M NaOH oder 1M H₂SO₄.

In Reaktor I wurden 213,2 ml deionisiertes Wasser, 15 ml einer 10 mM NAD⁺-Lösung, 200 ml eines 500 mM Kaliumphosphat-Puffers (pH 7), 50 ml ALDH I-Lysat sowie 0,5 g eines kommerziell erhältlichen NADH-Oxidase-Pulvers (NOX-009) unter Rührung vermischt und auf 35 °C temperiert. Zum Start der Reaktion wurden 20,5 g HMF (ca. 80% Reinheit) zugesetzt.

In Reaktor II wurden 213,2 ml deionisiertes Wasser, 15 ml einer 10 mM NAD⁺-Lösung, 200 ml eines 500 mM Kaliumphosphat-Puffers (pH 7), 50 ml ALDH I-Lysat sowie 0,5 g eines NADH-Oxidase-Pulvers (SEQ ID Nr. 14) unter Rührung vermischt und auf 35 °C temperiert. Zum Start der Reaktion wurden 20,5 g HMF (ca. 80% Reinheit) zugesetzt.

Zusätzlich wurde bei allen Reaktoren ein Überdruck von 320 mbar angelegt und die Sauerstoff-Zufuhr (über einen Sparger) wurde auf 0,05 l/min gestellt.

Zur Analyse wurden 50 µl eines Ansatzes mit 200 µl Acetonitril versetzt und im Eppendorf Thermomixer bei 85 °C und 1200 rpm für 15 min inkubiert. Die Probe wurde in einer Zentrifuge kurz zentrifugiert, mit 750 µl deionisiertem Wasser versetzt, gevortext und anschließend für 5 min bei max. g zentrifugiert. 250 µl des Überstands wurden in einem HPLC-Vial mit 750 µl einer Acetonitril/Wasser-Mischung (1/4 v/v) verdünnt und mittels HPLC (UV-Detektion) vermessen.

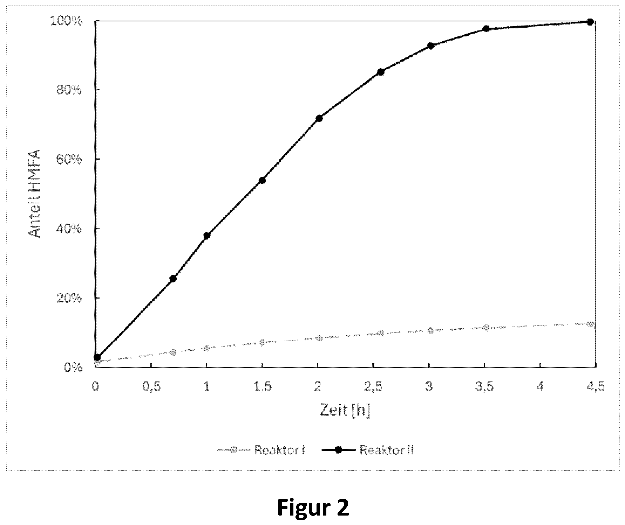

Die Ergebnisse sind als Konzentrationsverläufe in Figur 2 dargestellt. Sie zeigt, dass die Kofaktor-Regeneration mittels der NADH-Oxidase (SEQ ID Nr. 14) innerhalb von 4,5 h zu einem Umsatz von > 99% führt, während hingegen mit der kommerziellen NADH-Oxidase (NOX-009) nur ca. 13% Umsatz erreicht werden können.

### Beispiel 4

### Oxidation von 5-Hydroxymethylfurfural zu 2,5-Furandicarbonsäure via 5-Hydroxymethyl-2-furancarbonsäure (Fed-Batch-Verfahren)

Die Reaktion wurde in einem Labfors Tisch-Bioreaktor (Infors AG) durchgeführt. Als Gefäß wurde ein Glasreaktor (Volumen 3,4 l) mit aufgesetztem Rührwerk, O₂-Sensor und pH-Elektrode verwendet. Die pH-Kontrolle erfolgte durch die Zudosierung von 5M NaOH oder 1M H₂SO₄.

Zu Beginn wurden 212,7 ml deionisiertes Wasser, 200 ml eines 500 mM Kaliumphosphat-Puffers (pH 7), 15 ml einer 10 mM NAD⁺-Lösung, 50 ml ALDH I-Lysat sowie 21 kU NADH-Oxidase-Lysat vermischt und unter Rühren auf 30 °C gebracht. Zum Start der Reaktion wurden 20,5 g HMF (ca. 80% Reinheit) zugesetzt.

Zusätzlich wurde ein Überdruck von 320 mbar angelegt und die Sauerstoff-Zufuhr (über einen Sparger) wurde auf 0,05 l/min gestellt.

Zur Analyse wurden 50 µl der Reaktionsmischung mit 200 µl Acetonitril versetzt und im Eppendorf Thermomixer bei 85 °C und 1200 rpm für 15 min inkubiert. Die Probe wurde in einer Zentrifuge kurz zentrifugiert, mit 750 µl deionisiertem Wasser versetzt, gevortext und anschließend für 5 min bei max. g zentrifugiert. 200 µl des Überstands wurden in einem HPLC-Vial mit 800 µl einer Acetonitril/Wasser-Mischung (1/4 v/v) verdünnt und mittels HPLC (UV-Detektion) vermessen.

Innerhalb von 2 h wurde das Substrat HMF komplett zu HMFA oxidiert, daher wurden nach 2 h und ein weiteres Mal nach 4 h je 20,5 g HMF, 21 kU NADH-Oxidase-Lysat sowie 30 ml ALDH I-Lysat zugesetzt.

Nach 7 h wurden 50 ml HMFO-Lysat, 10 ml Katalase-Lysat, 30 ml ALDH I-Lysat sowie 21 kU NADH-Oxidase-Lysat zugesetzt. Nach 25 h wurden 50 ml HMFO-Lysat, 30 ml ALDH I-Lysat sowie 28 kU NADH-Oxidase-Lysat.

Auf diese Weise konnten in insgesamt 29 h > 99% des Substrats HMF zu FDCA oxidiert werden.

Danach wurde der Reaktorinhalt für 60 min auf 70 °C aufgeheizt. Der entstandene Niederschlag wurde durch Zentrifugation und Abdekantieren entfernt. Die Lösung wurde mit Aktivkohle (5% w/v) versetzt und 30 min bei 50 °C gerührt. Danach wurde die Aktivkohle über Celite in einer P4-Glasfritte abfiltriert. Das Filtrat wurde mit konzentrierter H₂SO₄ angesäuert (pH < 2) und auf 4 °C abgekühlt. Der entstandene Niederschlag (FDCA) wurde abfiltriert.

Auf diese Weise wurden 61 g FDCA als farbloser Feststoff gewonnen.

### Beispiel 5

### Oxidation von 5-Hydroxymethylfurfural zu 2,5-Furandicarbonsäure via 5-Hydroxymethyl-2-furancarbonsäure

Die Reaktion wurde in einem Labfors Tisch-Bioreaktor (Infors AG) durchgeführt. Als Gefäß wurde ein Glasreaktor (Volumen 3,4 l) mit aufgesetztem Rührwerk, O₂-Sensor und pH-Elektrode verwendet. Die pH-Kontrolle erfolgte durch die Zudosierung von 5M NaOH oder 1M H₂SO₄.

Zu Beginn wurden 304 ml deionisiertes Wasser, 100 ml eines 500 mM Kaliumphosphat-Puffers (pH 7), 15 ml einer 10 mM NAD⁺-Lösung, 50 ml ALDH I-Lysat sowie 21 kU NADH-Oxidase-Lysat vermischt und unter Rühren auf 30 °C gebracht. Zum Start der Reaktion wurden 31 g HMF (ca. 80% Reinheit) zugesetzt.

Zusätzlich wurde ein Überdruck von 320 mbar angelegt und die Sauerstoff-Zufuhr (über einen Sparger) wurde auf 0,05 l/min gestellt.

Zur Analyse wurden 50 µl der Reaktionsmischung mit 200 µl Acetonitril versetzt und im Eppendorf Thermomixer bei 85 °C und 1200 rpm für 15 min inkubiert. Die Probe wurde in einer Zentrifuge kurz zentrifugiert, mit 750 µl deionisiertem Wasser versetzt, gevortext und anschließend für 5 min bei max. g zentrifugiert. 200 µl des Überstands wurden in einem HPLC-Vial mit 800 µl einer Acetonitril/Wasser-Mischung (1/4 v/v) verdünnt und mittels HPLC (UV-Detektion) vermessen.

Nach 4,5 h wurden 50 ml ALDH I-Lysat und 21 kU NADH-Oxidase-Lysat zugesetzt.

Innerhalb von 7 h wurden > 99% des Substrats HMF zu HMFA oxidiert, daher wurden 50 ml HMFO-Lysat zugesetzt.

Nach 8 h wurden 50 ml ALDH I-Lysat sowie 21 kU NADH-Oxidase-Lysat und nach 11 h wurden 80 ml HMFO-Lysat zugesetzt.

Auf diese Weise konnten in insgesamt 23 h > 99% des Substrats HMF zu FDCA oxidiert werden.

### Beispiel 6

### Oxidation von 5-Hydroxymethylfurfural zu 2,5-Furandicarbonsäure via 5-Hydroxymethyl-2-furancarbonsäure - Kofaktor-Regeneration mit NADH-Oxidase und Alkoholdehydrogenase

Die Reaktion wurde in einem Labfors Tisch-Bioreaktor (Infors AG) durchgeführt. Als Gefäß wurde ein Glasreaktor (Volumen 3,4 l) mit aufgesetztem Rührwerk, O₂-Sensor und pH-Elektrode verwendet. Die pH-Kontrolle erfolgte durch die Zudosierung von 5M NaOH oder 1M H₂SO₄.

Zu Beginn wurden 212,7 ml deionisiertes Wasser, 200 ml eines 500 mM Kaliumphosphat-Puffers (pH 7), 15 ml einer 10 mM NAD⁺-Lösung, 50 ml ALDH I-Lysat sowie 21 kU NADH-Oxidase-Lysat vermischt und unter Rühren auf 35 °C gebracht. Zum Start der Reaktion wurden 20,5 g HMF (ca. 80% Reinheit) zugesetzt.

Zusätzlich wurde ein Überdruck von 320 mbar angelegt und die Sauerstoff-Zufuhr (über einen Sparger) wurde auf 0,05 l/min gestellt.

Zur Analyse wurden 50 µl der Reaktionsmischung mit 200 µl Acetonitril versetzt und im Eppendorf Thermomixer bei 85 °C und 1200 rpm für 15 min inkubiert. Die Probe wurde in einer Zentrifuge kurz zentrifugiert, mit 750 µl deionisiertem Wasser versetzt, gevortext und anschließend für 5 min bei max. g zentrifugiert. 200 µl des Überstands wurden in einem HPLC-Vial mit 800 µl einer Acetonitril/Wasser-Mischung (1/4 v/v) verdünnt und mittels HPLC (UV-Detektion) vermessen.

Innerhalb von 2,5 h wurden > 99% des Substrats HMF zu HMFA oxidiert.

Zur weiteren Oxidation wurden 30 ml HMFO-Lysat sowie 10 ml Katalase-Lysat zugesetzt. Nach 3,75 h wurden 50 ml ALDH I-Lysat, 30 ml Alkoholdehydrogenase-Lysat sowie 15 ml Aceton zugesetzt.

Innerhalb von 18 h wurden > 99% der HMFA zu FDCA oxidiert.

Auf diese Weise konnten in insgesamt 20,5 h > 99% des Substrats HMF zu FDCA oxidiert werden.

### Literatur

Carus, M., Dammer, L., Raschka, A., Skoczinski, P., & vom Berg, C. (2020). Renewable Carbon-Key to a Sustainable and Future-Oriented Chemical and Plastic Industry. https://renewable-carbon-initiative.com/wp-content/uploads/2020/09/20-09-21_Paper_12-on-Renewable-Carbon.pdf
Hamilton, L. A., Feit, S., Muffett, C., Kelso, M., Rubright, S. M., Bernhardt, C., Schaeffer, E., Moon, D., Morris, J., & Labbé-Bellas, R. (2019). Plastic & Climate: The Hidden Costs of a Plastic Planet. https://www.ciel.org/wp-content/uploads/2019/05/Plastic-and-Climate-FINAL-2019.pdf
Tomäs, R. A. F., Bordado, J. C. M., & Gomes, J. F. P. (2013). p-Xylene Oxidation to Terephthalic Acid: A Literature Review Oriented toward Process Optimization and Development. Chemical Reviews, 113(10), 7421-7469. https://doi.org/10.1021/cr300298j
Berger, A. (2016). Ethylenglycol, RD-05-01999 in Böckler, F., Dill, B., Eisenbrand, G., Faupel, F., Fugmann, B., Gamse, T., Matissek, R., Pohnert, G., Rühling, A., Schmidt, S., Sprenger, G. (Ed.), RÖMPP [Online], Stuttgart, Georg Thieme Verlag. https://roempp.thieme.de/lexicon/RD-05-01999
Fan, D., Dai, D.-J., & Wu, H.-S. (2013). Ethylene Formation by Catalytic Dehydration of Ethanol with Industrial Considerations. Materials 6(1), 101-115. https://doi.org/10.3390/ma6010101
Salusjärvi, L., Havukainen, S., Koivistoinen, O., & Toivari, M. (2019). Biotechnological production of glycolic acid and ethylene glycol: current state and perspectives. Applied Microbiology and Biotechnology, 103, 2525-2535. https://doi.org/10.1007/s00253-019-09640-2
de Jong, E., Visser, H. A., Dias, A. S., Harvey, C., & Gruter, G.-J. M. (2022). The Road to Bring FDCA and PEF to the Market. Polymers, 14(5), 943. https://doi.org/10.3390/polym14050943
Cong, H., Yuan, H., Tao, Z., Bao, H., Zhang, Z., Jiang, Y., Huang, D., Liu, H., & Wang, T. (2021). Recent Advances in Catalytic Conversion of Biomass to 2,5-Furandicarboxylic Acid. Catalysts, 11(9), 1113. https://doi.org/10.3390/catal11091113
Todea, A., Bitcan, I., Aparaschivei, D., Päusescu, I., Badea, V., Péter, F., Gherman, V. D., Rusu, G., Nagy, L., & Kéki, S. (2019). Biodegradable Oligoesters of ε-Caprolactone and 5-Hydroxymethyl-2-Furancarboxylic Acid Synthesized by Immobilized Lipases. Polymers, 11(9), 1402. https://doi.org/10.3390/polym11091402
Munekata, M., & Tamura, G. (1981). Antitumor Activity of 5-Hydroxymethyl-2-furoic Acid. Agricultural and Biological Chemistry, 45(9), 2149-2150. https://doi.org/10.1080/00021369.1981.10864851
Kimura, Y., Tani, S., Hayashi, A., Ohtani, K., Fujioka, S., Kawano, T., & Shimada, A. (2007). Nematicidal Activity of 5-Hydroxymethyl-2-furoic Acid against Plant-Parasitic Nematodes. Zeitschrift für Naturforschung C, 62(3-4), 234-238. https://doi.org/10.1515/znc-2007-3-413
Cang, R., Shen, L.-Q., Yang, G., Zhang, Z.-D., Huang, H., & Zhang, Z.-G. (2019). Highly Selective Oxidation of 5-Hydroxymethylfurfural to 5-Hydroxymethyl-2-Furancarboxylic Acid by a Robust Whole-Cell Biocatalyst. Catalysts, 9(6), 526. https://doi.org/10.3390/catal9060526
Hossain, G. S., Yuan, H., Li, J., Shin, H., Wang, M., Du, G., Chen, J., & Liu, L. (2017). Metabolic Engineering of Raoultella ornithinolytica BF60 for Production of 2,5-Furandicarboxylic Acid from 5-Hydroxymethylfurfural. Applied and Environmental Microbiology, 83(1), e02312-16. https://doi.org/10.1128/AEM.02312-16
Zhang, X.-Y., Wang, X., Li, N.-W., Guo, Z.-W., Zong, M.-H., & Li, N. (2020). Furan Carboxylic Acids Production with High Productivity by Cofactor-engineered Whole-cell Biocatalysts. ChemCatChem, 12(12), 3257-3264. https://doi.org/10.1002/cctc.202000259
Knaus, T., Tseliou, V., Humphreys, L. D., Scrutton, N. S., & Mutti, F. G. (2018). A biocatalytic method for the chemoselective aerobic oxidation of aldehydes to carboxylic acids. Green Chemistry, 20(17), 3931-3943. https://doi.org/10.1039/C8GC01381K
Qin, Y.-Z., Li, Y.-M., Zong, M.-H., Wu, H., & Li, N. (2015). Enzyme-catalyzed selective oxidation of 5-hydroxymethylfurfural (HMF) and separation of HMF and 2,5-diformylfuran using deep eutectic solvents. Green Chemistry, 17(7), 3718-3722. https://doi.org/10.1039/C5GC00788G
McKenna, S. M., Leimkühler, S., Herter, S., Turner, N. J., & Carnell, A. J. (2015). Enzyme cascade reactions: Synthesis of furandicarboxylic acid (FDCA) and carboxylic acids using oxidases in tandem. Green Chemistry, 17(6), 3271-3275. https://doi.org/10.1039/C5GC00707K
McKenna, S. M., Mines, P., Law, P., Kovacs-Schreiner, K., Birmingham, W. R., Turner, N. J., Leimkühler, S., & Carnell, A. J. (2017). The continuous oxidation of HMF to FDCA and the stabilization of periplasmic aldehyde oxidase (PaoABC). Green Chemistry, 19(19), 4660-4665. https://doi.org/10.1039/C7GC01696D
Carro, J., Ferreira, P., Rodriguez, L., Prieto, A., Serrano, A., Balcells, B., Ardá, A., Jiménez-Barbero, J., Gutiérrez, A., Ullrich, R., Hofrichter, M., & Martinez, A. T. (2015). 5-hydroxymethylfurfural conversion by fungal aryl-alcohol oxidase and unspecific peroxygenase. FEBS Journal, 282(16), 3218-3229. https://doi.org/10.1111/febs.13177
Mathieu, Y., Offen, W. A., Forget, S. M., Ciano, L., Viborg, A. H., Blagova, E., Henrissat, B., Walton, P. H., Davies, G. J., & Brumer, H. (2020). Discovery of a Fungal Copper Radical Oxidase with High Catalytic Efficiency toward 5-Hydroxymethylfurfural and Benzyl Alcohols for Bioprocessing. ACS Catalysis, 10(5), 3042-3058. https://doi.org/10.1021/acscatal.9b04727
Cleveland, M., Lafond, M., Xia, F. R., Chung, R., Mulyk, P., Hein, J. E., & Brumer, H. (2021). Two Fusarium copper radical oxidases with high activity on aryl alcohols. Biotechnology for Biofuels, 14, 138. https://doi.org/10.1186/s13068-021-01984-0
Cajnko, M. M., Novak, U., Grilc, M., & Likozar, B. (2020). Enzymatic conversion reactions of 5-hydroxymethylfurfural (HMF) to bio-based 2,5-diformylfuran (DFF) and 2,5-furandicarboxylic acid (FDCA) with air: mechanisms, pathways and synthesis selectivity. Biotechnology for Biofuels, 13, 66. https://doi.org/10.1186/s13068-020-01705-z
Lappe, A., Jankowski, N., Albrecht, A., Koschorreck, K. (2021). Characterization of a thermotolerant aryl-alcohol oxidase from Moesziomyces antarcticus oxidizing 5-hydroxymethyl-2-furancarboxylic acid. Applied Microbiology and Biotechnology, 105, 8313-8327. https://doi.org/10.1007/s00253-021-11557-8
Jia, H.-Y., Zong, M.-H., Yu, H.-L., & Li, N. (2017). Dehydrogenase-Catalyzed Oxidation of Furanics: Exploitation of Hemoglobin Catalytic Promiscuity. ChemSusChem, 10(18), 3524-3528. https://doi.org/10.1002/cssc.201701288
Altschul, S. F., Gish, W., Miller, W., Myers, E. W., & Lipman, D. J. (1990). Basic local alignment search tool. Journal of Molecular Biology, 215(3), 403-410. https://doi.org/10.1016/S0022-2836(05)80360-2
Henikoff, S., & Henikoff, J. G. (1992). Amino acid substitution matrices from protein blocks. Proceedings of the National Academy of Sciences of the United States of America, 89(22), 10915-10919. https://doi.org/10.1073/pnas.89.22.10915
Sambrook, J., Fritsch, E. R., & Maniatis, T. (1989). Molecular Cloning: A Laboratory Manual (2nd ed.). Cold Spring Harbor, NY: Cold Spring Harbor Laboratory Press.
Lin, B., & Tao, Y. (2017). Whole-cell biocatalysts by design. Microbial Cell Factories, 16, 106. https://doi.org/10.1186/s12934-017-0724-7
Protein [Internet]. Bethesda (MD): National Library of Medicine (US), National Center for Biotechnology Information. Accession No. WP_024766379.1, aldehyde dehydrogenase family protein [Pseudomonas nitroreducens]. Verfügbar unter: https://www.ncbi.nlm.nih.gov/protein/WP_024766379.1/
Protein [Internet]. Bethesda (MD): National Library of Medicine (US), National Center for Biotechnology Information. Accession No. WP_015829138.1, aldehyde dehydrogenase family protein [Methylovorus glucosotrophus]. Verfügbar unter: https://www.ncbi.nlm.nih.gov/protein/WP_015829138.1/
Protein [Internet]. Bethesda (MD): National Library of Medicine (US), National Center for Biotechnology Information. Accession No. WP_169935101.1, aldehyde dehydrogenase family protein [Pseudomonas multiresinivorans]. Verfügbar unter: https://www.ncbi.nlm.nih.gov/protein/WP_169935101.1
Protein [Internet]. Bethesda (MD): National Library of Medicine (US), National Center for Biotechnology Information. Accession No. WP_004866732.1, MULTISPECIES: aldehyde dehydrogenase family protein [Raoultella]. Verfügbar unter: https://www.ncbi.nlm.nih.gov/protein/ WP_004866732.1
Protein [Internet]. Bethesda (MD): National Library of Medicine (US), National Center for Biotechnology Information. Accession No. WP_003076354.1, MULTISPECIES: aldehyde dehydrogenase [Comamonas]. Verfügbar unter: https://www.ncbi.nlm.nih.gov/protein/WP_003076354.1
Protein [Internet]. Bethesda (MD): National Library of Medicine (US), National Center for Biotechnology Information. Accession No. WP_011015386.1, aldehyde dehydrogenase family protein [Corynebacterium glutamicum]. Verfügbar unter: https://www.ncbi.nlm.nih.gov/protein/WP_011015386.1
Protein [Internet]. Bethesda (MD): National Library of Medicine (US), National Center for Biotechnology Information. Accession No. WP_024766380.1, GMC oxidoreductase [Pseudomonas nitroreducens]. Verfügbar unter: https://www.ncbi.nlm.nih.gov/protein/WP_024766380.1
Sakoda, H., & Imanaka, T. (1992). Cloning and sequencing of the gene coding for alcohol dehydrogenase of Bacillus stearothermophilus and rational shift of the optimum pH. Journal of Bacteriology, 174(4), 1397-1402. https://doi.org/10.1128/jb.174.4.1397-1402.1992
Protein [Internet]. Bethesda (MD): National Library of Medicine (US), National Center for Biotechnology Information. Accession No. WP_067978261.1, FAD-dependent oxidoreductase [Aerococcus urinaehominis]. Verfügbar unter: https://www.ncbi.nlm.nih.gov/protein/WP_067978261.1/
Protein [Internet]. Bethesda (MD): National Library of Medicine (US), National Center for Biotechnology Information. Accession No. WP_092479712.1, FAD-dependent oxidoreductase [Desemzia incerta]. Verfügbar unter: https://www.ncbi.nlm.nih.gov/protein/WP_092479712.1
UniProt [Internet]. Accession No. P29422, CATA_MICLU (Catalase from Micrococcus luteus). Verfügbar unter: https://www.uniprot.org/uniprotkb/P29422/entry
Viñambres, M., Espada, M., Martinez, A. T., & Serrano, A. (2020). Screening and Evaluation of New Hydroxymethylfurfural Oxidases for Furandicarboxylic Acid Production. Applied and Environmental Microbiology, 86(16), e00842-20. https://doi.org/10.1128/AEM.00842-20

## Patentansprüche

1. Verfahren zur Herstellung von 5-Hydroxymethyl-2-furancarbonsäure (HMFA), indem 5-Hydroxymethylfurfural (HMF), welches in einer wässrigen Lösung vorliegt, durch Behandeln mit einer NAD(P)⁺-abhängigen Aldehyd-Dehydrogenase *in vitro* unter Bildung von NAD(P)H zu 5-Hydroxymethyl-2-furancarbonsäure (HMFA) oxidiert wird, wonach das bei der Oxidation entstandene NAD(P)H enzymatisch mit einer NAD(P)H-Oxidase wieder zu NAD(P)⁺ regeneriert wird, **dadurch gekennzeichnet, dass** die NAD(P)H-Oxidase eine Aminosäuresequenz umfasst, welche ausgewählt ist aus der Gruppe bestehend aus:
i) einer Aminosäuresequenz, die eine Identität zu SEQ ID Nr. 14, SEQ ID Nr. 16 oder SEQ ID Nr. 18 von mindestens 80% aufweist,
ii) einer Aminosäuresequenz, die durch eine Nukleinsäure kodiert wird, die eine Identität zu SEQ ID Nr. 13, SEQ ID Nr. 15 oder SEQ ID Nr. 17 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 13, SEQ ID Nr. 15 oder SEQ ID Nr. 17 bindet.

2. Verfahren zur Herstellung von 2,5-Furandicarbonsäure, **dadurch gekennzeichnet, dass** in dem Verfahren gemäß Anspruch 1 der wässerigen Lösung nach Bildung von 5-Hydroxymethyl-2-furancarbonsäure (HMFA) eine 5-Hydroxymethylfurfural-Oxidase zugegeben wird.

3. Verfahren nach Anspruch 2, **dadurch gekennzeichnet, dass** der wässerigen Lösung auch eine Katalase zugegeben wird.

4. Verfahren nach einem der Ansprüche 1 bis 3, **dadurch gekennzeichnet, dass** das bei der Oxidation entstandene NAD(P)H zumindest zum Teil enzymatisch mit einer Oxidoreduktase wieder zu NAD(P)⁺ regeneriert und als Kosubstrat der Oxidoreduktase eine Keto-Verbindung eingesetzt wird.

5. Verfahren nach Anspruch 4, **dadurch gekennzeichnet, dass** als Keto-Verbindung eine Ketose, eine Aldose oder ein Keton eingesetzt wird.

6. Verfahren nach Anspruch 5, **dadurch gekennzeichnet, dass** als Ketose oder Aldose D-Fructose, D-Glucose oder D-Xylose eingesetzt wird.

7. Verfahren nach Anspruch 5, **dadurch gekennzeichnet, dass** als Keton ein aliphatisches Keton eingesetzt wird.

8. Verfahren nach Anspruch 7, **dadurch gekennzeichnet, dass** als aliphatisches Keton Aceton eingesetzt wird.

9. Verfahren nach einem der Ansprüche 1 bis 8, **dadurch gekennzeichnet, dass** die NAD(P)⁺-abhängige Aldehyd-Dehydrogenase eine Aminosäuresequenz aufweist, welche ausgewählt ist aus der Gruppe bestehend aus:
i) einer Aminosäuresequenz, die eine Identität zu SEQ ID Nr. 2, SEQ ID Nr. 4, SEQ ID Nr. 6, SEQ ID Nr. 8, SEQ ID Nr. 10 oder SEQ ID Nr. 12 von mindestens 80% aufweist,
ii) einer Aminosäuresequenz, die durch eine Nukleinsäure kodiert wird, die eine Identität zu SEQ ID Nr. 1, SEQ ID Nr. 3, SEQ ID Nr. 5, SEQ ID Nr. 7, SEQ ID Nr. 9 oder SEQ ID Nr. 11 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 1, SEQ ID Nr. 3, SEQ ID Nr. 5, SEQ ID Nr. 7, SEQ ID Nr. 9 oder SEQ ID Nr. 11 bindet.
